(19) Europäisches Patentamt
European Patent Office
Office européen des brevets

(11) **EP 1 465 990 B1**

(12) **EUROPEAN PATENT SPECIFICATION**

(45) Date of publication and mention of the grant of the patent:
**13.07.2011 Bulletin 2011/28**

(21) Application number: **02806841.9**

(22) Date of filing: **23.12.2002**

(51) Int Cl.:
*C12N 15/63* (2006.01) *C12N 9/50* (2006.01)
*A61K 38/48* (2006.01) *A61P 31/04* (2006.01)

(86) International application number:
**PCT/US2002/040924**

(87) International publication number:
**WO 2003/082184 (09.10.2003 Gazette 2003/41)**

(54) **TRUNCATED LYSOSTAPHIN MOLECULE WITH ENHANCED STAPHYLOLYTIC ACTIVITY**

GESTUTZES LYSOSTAPHIN-MOLEKÜL MIT VERBESSERTER STAPHYLOLYTISCHER WIRKUNG

MOLECULE DE LYSOSTAPHINE TRONQUEE A ACTIVITE STAPHYLOLYTIQUE AMELIOREE

(84) Designated Contracting States:
**AT BE BG CH CY CZ DE DK EE ES FI FR GB GR IE IT LI LU MC NL PT SE SI SK TR**

(30) Priority: **21.12.2001 US 341804 P**

(43) Date of publication of application:
**13.10.2004 Bulletin 2004/42**

(73) Proprietor: **Biosynexus Incorporated**
**Gaithersburg, MD 20877 (US)**

(72) Inventors:
- **STINSON, Jeffrey, R.**
  **Brookeville, MD 20833 (US)**
- **GRINBERG, Lioubov**
  **Gaithersburg, MD 20878 (US)**
- **LEES, Andrew**
  **Silver Spring, MD 20910 (US)**
- **MOND, James, J.**
  **Silver Springs, MD 20901 (US)**
- **KOKAI-KUN, John, F.**
  **Frederick, MD 21703 (US)**

(74) Representative: **Baldock, Sharon Claire et al**
**Boult Wade Tennant**
**Verulam Gardens**
**70 Gray's Inn Road**
**London WC1X 8BT (GB)**

(56) References cited:
**EP-A- 0 759 473** **WO-A-87/06264**
**WO-A-99/05289** **WO-A-99/67381**
**WO-A1-01/29201** **WO-A1-99/05289**
**WO-A2-01/85959** **US-A- 5 011 772**

- **CAVADINI C, HERTEL C, HAMMES W P: "Stable Expression of the Lysostaphin Gene in Meat Lactobacilli by Introducing Deletions within the Prosequnce" SYSTEM. APPL. MICROBIOL., vol. 19, 1996, pages 21-27, XP009066714**
- **THUMM ET AL: 'Studies on prolysostaphin processing and characterization of the lysostaphin immunity factor (LIf) of Staphylococcus simulans biovar staphylolyticus' MOLECULAR MICROBIOLOGY vol. 23, no. 6, 1997, pages 1251 - 1265, XP002921139**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 465 990 B1

## Description

## INTRODUCTION

Lysostaphin is an antibacterial enzyme first identified in *Staphylococcus simulans* (formerly known as *S. staphylolyticus*) in 1964. Lysostaphin is an endopeptidase capable of specifically cleaving the cross-linking pentaglycine bridges in the cell walls of staphylococci (27). Expressed in a single polypeptide chain, lysostaphin has a molecular weight of Approximately 27 KDa

Because the cell wall bridges of *Staphylococcus aureus* contain a high proportion of glycine, lysostaphin is particularly effective in lysing *S. aureus,* a coagulase positive staphylococcus (27). Initial studies with lysostaphin also demonstrated that this enzyme could lyse *Staphylococcus epidermidis,* a coagulase negative staphylococcus. Subsequent studies found, however, that in comparison to *S. aureus,* lysing *S. epidermidis* required either higher concentrations of enzyme or longer incubation times depending on the strain of *S. epidermidis* (27). This strain-specific sensitivity to lysostaphin in *S. epidermidis* is thought to be due to differing amounts of glycine in the cell walls of each strain. Those strains that are more resistant to lysostaphin contain a higher proportion of serine in the cell wall rather than glycine (27).

Staphylococcal infections, such as those caused by *S. aureus,* are a significant cause of morbidity and mortality, particularly in settings such as hospitals, schools, and infirmaries. Patients particularly at risk include infants, the elderly, the immunocompromised, the immunosuppressed, and those with chronic conditions requiring frequent hospital stays. Further, the advent of multiple drug resistant strains of *Staphylococcus aureus* increases the concern and need for timely blocking and treatment of such infections. Indeed, the recent World Health Organization report entitled "Overcoming Astionicro Oral Resistance" detailed its concern that increasing levels of drug resistance are threatening to erode the medical advances of the recent decades. Among the issues raised are infections in hospitalized patients. In the United States alone, some 14,000 people are infected and die each year as a result of drug-resistant microbes acquired in hospitals. Around the world, as many as 60% of hospital-acquired infections are caused by drug-resistant microbes.

Patients at greatest risk are those undergoing inpatient or outpatient surgery, in the Intensive Care Unit (ICU), on continuous hemodialysis, with HIV infection, with AIDS, burn victims, people with diminished natural immunity from treatments or disease, chronically ill or debilitated patients, geriatric populations, infants with immature immune systems, and people with intravascular devices (2, 3, 4, 7, 8, 9, 13, 25, 26).

In infections caused by *S. aureus,* it appears that a principal ecological niche for *S. aureus* is the human anterior nares. Nasal carriage of staphylococci plays a key role in the epidemiology and pathogenesis of infection (2, 3, 7, 13, 23, 24, 25, 26). In healthy subjects, three patterns of *S. aureus* nasal carriage can be distinguished over time: approximately 20% of people are persistent carriers, approximately 60% are intermittent carriers, and approximately 20% apparently never carry *S. aureus* (7).

Chang et al. (1) studied 84 patients with cirrhosis admitted to a liver transplant unit. Overall, 39 (46%) were nasal carriers of *S. aureus* and 23% of these patients subsequently developed *S. aureus* infections as compared to only 4% of the non-carriers. A study of HIV patients (13) showed that 49% (114 of 296) of patients had at least one positive nasal culture for *S. aureus*. Thirty four percent of 201 patients were considered nasal carriers, with 38% of these being persistent carriers, and 62% intermittent carriers. Twenty-one episodes of *S. aureus* infection occurred in thirteen of these patients. Molecular strain typing indicated that, for six of seven infected patients, the strain of *S. aureus* isolated from the infected site was the same as that previously cultured from the nares, underlining the need for blocking of even apparently benign nasal colonization. The nasal *S. aureus* carrier patients were significantly more likely to develop *S. aureus* infection (P=0.04; odds ratio, 3.6; attributable risk, 0.44). This finding led the authors to conclude that nasal carriage is an important risk factor for *S. aureus* infection in HIV patients (13).

In a related patent application, *Methods and Formulations for Eradicating or Alleviating Staphylococcal Nasal Colonization Using Lysostaphin,* submitted concurrently herewith and specifically incorporated by reference, lysostaphin was shown to be effective in blocking and alleviating colonization of the nose by *S. aureus* when applied directly to the anterior nares in a viscous formulation. This lysostaphin formulation was effective against nasal colonization when applied concurrently with *S. aureus* in a cotton rat model or after nasal colonization was established in this animal model.

Lysostaphin may also provide therapy against active *S. aureus* infections of the skin, blood, and solid tissues. Among these are treatments for endocarditis (28) and systemic *S. aureus* (29) infections.

Given that (1) *S. aureus* infections are prevalent in the general population; (2) *S. aureus* strains resistant to current antibiotics are emerging in the general population; (3) lysostaphin is very active against *S. aureus* including mutli-drug resistant strains and (4) a lysostaphin viscous formulation is effective in blocking and alleviating colonization in the nose (a major reservoir for *S. aureus* infection), there is a need in the art for a means of producing large quantities of lysostaphin for use in blocking and alleviating staphylococcal infections and colonization.

## BRIEF DESCRIPTION OF THE INVENTION

This invention relates to recombinant truncated lysostaphin. The lysostaphin of the invention is for use in patients at risk for staphylococcal infection. Populations at risk include infants with immature immune systems, patients admitted to the hospital for in-patient or out-patient surgical procedures, patients suffering from various conditions that predispose them to staphylococcal colonization and/or infections, or any patient prior to release from a hospital. The use of lysostaphin intranasally as a pre-release treatment will serve to reduce individual infections as well as to inhibit community spread of hospital-acquired staphylococcal strains.

The lysostaphins of the invention may be administered by application to skin, wounds, eyes, ears, lungs, mucus membranes of the nasal or gastrointestinal tracts. The lysostaphins may be administered by inhalation or other instillation into the nares or anterior nares of a patient.

Lysostaphin may be administered to a patient in several forms. Lysostaphin may be added to a viscous cream or liquid formulation. Viscous creams or liquids are acceptable for for intranasal administration and administration by other routes. Other possible carriers comprise natural polymers, semi-synthetic polymers, synthetic polymers, liposomes, and semi-solid dosage forms (10, 11, 12, 14, 16, 19, 21, 22). Natural polymers include, for example, proteins and polysaccharides. Semi-synthetic polymers are modified natural polymers such as chitosan, which is the deacetylated form of the natural polysaccharide, chitin. Synthetic polymers include, for example, dedrimers, polyphosphoesters, polyethylene glycol, poly (lactic acid), polystyrene sulfonate, and poly (lactide coglycolide). Semi-solid dosage forms include, for example, creams, ointments, gels, and lotions.

The invention includes compositions comprised of recombinant truncated lysostaphin. Recombinant DNA molecules that encode one homogenous form of lysostaphin, which may or may not be truncated, host cells transformed with these DNA molecules, and methods of producing lysostaphin from these transformed cells are also described. Lysostaphin expressed from the recombinant DNA molecule accumulates inside the transformed host cell in its final form. Lysostaphin expressed from the recombinant DNA molecule is secreted into the host cell media in its final form. Thus, the lysostaphin molecules produced by the methods described herein do not require proteolysis to reach their final state. The methods described herein may also be applied to any truncated or mutated functional form of lysostaphin to achieve expression and production of a homogenous population of lysostaphin molecules.

## BRIEF DESCRIPTION OF THE DRAWINGS

Figure 1 provides a diagram summarizing the method of overlap extension PCR cloning.

Figure 2 provides a restriction map of plasmid pJSB28, the amino acid sequence of the truncated lysostaphin protein encoded by pJSB28, and the nucleotide sequence encoding the truncated lysostaphin protein in pJSB28.

Figure 3 provides a restriction map of plasmid pJSB20, the amino acid sequence of the truncated lysostaphin protein and signal polypeptide encoded by pJSB20, and the nucleotide sequence encoding the truncated lysostaphin protein and signal polypeptide in pJSB20. Underlined amino acids indicate the signal polypeptide sequence.

Figure 4 demonstrates via an ELISA assay that host cells transformed with pJSB28 or pJSB20 produce intracellular and extracellular truncated lysostaphin, respectively.

Figure 5 demonstrates via a *S. aureus* lysing assay that host cells transformed with pJSB28 or pJSB20 produce functional truncated lysostaphin.

Figures 6A and 6B demonstrate via a colorimetric assay that the truncated form of lysostaphin is more active than the mature full length form.

Figures 7A - 7D respectively provide restriction maps of the plasmids pRN5550, pNZ8148ScaI, pLSS1C, and pLss12F. pLss12F encodes a truncated form of lysostaphin that has been bulk purified using methods disclosed herein.

Figure 8 provides the nucleic acid sequence encoding truncated lysostaphin found in the plasmid pLss12F. The nucleic acid sequence contains a silent T to C mutation at position 255.

Figure 9 shows the truncated form of lyphostatin on an SDS-PAGE reducing gel following the bulk purification protocol.

Figures 10-12 compare the bacteriocidal activity of homogeneous truncated and homogeneous mature lyphostaphins over a range of lysostaphin concentrations.

Figures 13 and 14 compare the bacteriocidal activity of homogeneous truncated and homogeneous mature lyphostaphins over a 60 minute time course in cultured human blood.

Figures 15 and 16 compare the bacteriocidal activity of homogeneous truncated and homogeneous mature lyphostaphins in an OD-drop assay.

## DETAILED DESCRIPTION OF THE INVENTION

Lysostaphin is naturally produced by bacteria as a pro-enzyme that is cleaved to produce the mature form of lysostaphin. The pro-enzyme contains a signal sequence for secretion of the enzyme, a tandem, repetitive motif typically consisting of 7 repeats of 13 amino acids, and the final full length (mature) protein of 246 amino acids. The enzyme that cleaves pro-lysostaphin is inconsistent in where it cleaves the molecule. But a lysostaphin molecule that has been incorrectly cleaved may be cleaved again by the same enzyme until the final form, beginning with amino acids "AATHE," is produced (SEQ ID NO: 17). Accordingly, those in the art have agreed that mature, full length lysostaphin begins at the amino acid directly following the pro-sequence that is normally cleaved. Thus, mature lysostaphin begins with the amino acid sequence, "AATHE...." When lysostaphin is isolated from bacteria, a snap shot of all the forms of lysostaphin present in the bacteria at the time of isolation results. Several forms of lysostaphin are present in the resulting preparation: 1) less active pro-lysostaphin; 2) active mature lysostaphin ("AATHE" form); and 3) intermediate forms of lysostaphin, i.e., molecules at various stages of maturation that have different amounts of pro-sequence not yet cleaved from the molecule. Thus, preparations from natural sources result in a heterologous population of active and less active lysostaphins that begin at different amino acids.

The presence of less active forms of lysostaphin dilutes out the concentration of fully active lysostaphin in the preparation, thus decreasing the specific activity of a composition containing naturally derived lysostaphin. In contrast, recombinant lysostaphin preparations contain only a fully active, mature or truncated form of lysostaphin. In such preparations, there is no less active form to dilute out the activity of the recombinant molecules. Thus, the specific activity of a composition made with recombinant lysostaphin will be higher than one made with naturally-derived lysostaphin.

Under current production methodologies, the lysostaphin is expressed in *B. sphaericus,* from a genetically engineered plasmid, as a pro-protein that is post-translationally processed to its full length active form. The active enzyme is then isolated from the growth medium. As with natural forms of lysostaphin, this product, however, consists of a mixture of polypeptides ranging from 248 to 244 amino acids in length due to differential proteolysis of the pro-enzyme (5). As such, lysostaphin sequences, for example those in Ambicin L (Ambi, Purchase NY), may begin with the sequences "RAATHE...," "LRAATHE...," "AATHE...," or "THE...." (SEQ ID NOS: 15-17; SEQ ID NO: 13).

One aspect of the invention relates to compositions comprising homogenous recombinant truncated lysostaphin. In one aspect of this embodiment, the gene for lysostaphin was genetically truncated to remove the lysostaphin signal sequence, the repetitive elements (the "pro" domain), and the first two alanine amino acids in the full length lysostaphin amino acid sequence. This truncated lysostaphin sequence, beginning with the amino acids "THE," was fused to either an initiating methionine for intracellular expression or a signal sequence allowing the secretion of a single species of truncated lysostaphin into the periplasmic space of *E. coli*. In removing the first two amino acids of the mature lysostaphin sequence (ala-ala), the inventors have generated a form of lysostaphin with improved antistapylococcal activity as compared to the mature full length protein. Other embodiments may include other truncations.

In other aspects of the invention, homogenous truncated lysostaphin may be used in a pharmaceutical composition for treatment of staphylococcal nasal colonization or as a therapy for various active *S. aureus* infections. Government standards often require that an ingredient added to a pharmaceutical composition must be a homogenous form. A mixture of different chemical forms of the ingredient is less likely to be incorporated into a pharmaceutical composition. Thus, the homogenous, truncated lysostaphin of the invention is well-suited for use in pharmaceutical compositions.

The present invention pharmaceutical compositions comprises a therapeutically effective amount of a lysphostatin of the invention, together with a pharmaceutically acceptable carrier. Pharmaceutically acceptable carriers can be sterile liquids, such as water, oils, including petroleum oil, animal oil, vegetable oil, peanut oil, soybean oil, mineral oil, sesame oil, and the like. Saline solutions, aqueous dextrose, and glycerol solutions can also be employed as liquid carriers, particularly for injectable solutions. Suitable pharmaceutical carriers are described in *Remington's Pharmaceutical Sciences, 18th Edition* (13).

A therapeutically effective amount is an amount reasonably believed to provide some measure of relief, assistance, prophylaxis, or preventative effect in the treatment of the infection. A therapeutically effective amount may be an amount believed to be sufficient to block a bacterial colonization or infection. Similarly, a therapeutically effective amount may be an amount believed to be sufficient to alleviate an existing bacterial infection.

Methods for the production of homogenous lysostaphin are also provided. Unlike the current production methods discussed above, the method described herein is designed to express lysostaphin proteins all of which begin at the same amino acid by removing the need for proteolysis after expression of the recombinant protein. The method described herein uses DNA molecules, discussed below, that express lysostaphin in its final form, whether full length or truncated. Because the resulting lysostaphin molecules do not require unpredictable proteolysis to reach their final form, the instant method results in a substantially homogenous preparation of lysostaphin with each molecule beginning at the same amino acid. The homogenous lysostaphin of the invention also carries with it the added advantage of simplifying detection of contaminants. Specifically, contaminants from the bacterial cells expressing lysostaphin may remain in the preparation. Finding or eliminating those contaminants in a preparation is more difficult when several forms of lysostaphin are present in the preparation, given the required chemical analysis to test for purity. Thus, a system that expresses homogenous lysostaphin is more readily adaptable to producing pure lysostaphin, free of contaminants, for potential clinical use.

A recombinant DNA molecule encoding a homogenous form of lysostaphin is also provided. The recombinant DNA molecule may be a plasmid that is comprised of an origin of replication, a gene conferring antibiotic resistance to the transformed host cell, a promoter region operatively linked to a DNA sequence encoding lysostaphin, a signal sequence, and a termination sequence. The recombinant DNA molecule may also be in an alternate form such as a cosmid or linearized DNA. The recombinant DNA may also be incorporated into the host cell genome for purposes of stability during commercial, pharmaceutical production. The recombinant DNA molecule may encode full length lysostaphin, truncated lysostaphin, or a lysostaphin variant. The recombinant DNA molecule described herein may encode lysostaphin that localizes inside the cell (i.e., intracellular expression) or may encode lysostaphin with a signal for secretion of lysostaphin into the host cell media (i.e., extracellular expression).

The recombinant DNA molecule described herein also includes DNA molecules that do not express lysostaphin protein, but that contain a nucleotide sequence that is capable of expressing lysostaphin protein if operably linked to appropriate genetic control elements (i.e., a promoter).

The term "lysostaphin," as used herein, means full length lysostaphin, any lysostaphin mutant or variant, any lysostaphin truncation, any recombinantly expressed lysostaphin protein, or a related enzyme that retains the proteolytic ability, *in vitro* and *in vivo,* of proteolytic attack against glycine-containing bridges in the cell wall peptidoglycan of staphylococci. Modified full-length lysostaphin or lysostaphin variants may be generated by post-translational processing of the protein (either by enzymes present in a host cell strain or by means of enzymes or reagents introduced at any stage of the process) or by mutation of the structural gene.

Mutations may include deletion, insertion, domain removal, point and replacement mutations. Lysostaphin includes, for example, lysostaphin purified from *S. simulans,* Ambicin L (Nutrition 21, Inc.), purified from *B. sphaericus,* or lysostaphin purified from a recombinant expression system such as the instant invention. Truncated lysostaphin includes any lysostaphin protein in which one or more amino acids have been removed from the protein's amino terminus, carboxy terminus, or both. Lysostaphin variants may also be expressed in a truncated form.

The term "express," as used herein, refers to the process by which messenger RNA is transcribed from a DNA template such that the messenger RNA is then translated into the amino acid sequence that forms a protein. Thus, a DNA molecule expresses lysostaphin when it contains nucleotide sequences that may be transcribed into messenger RNA that will be translated into a lysostaphin protein. For the purposes of this invention, the term "express" is essentially equivalent to the term "functionally encode."

The term "origin of replication," as used herein, means a DNA sequence that allows an extrachromosomal piece of DNA, such as a plasmid, to duplicate itself independently of chromosomal replication. The origin of replication often binds host cell proteins that participate in DNA replication in the cell.

The term "promoter," as used herein, refers to a DNA sequence that facilitates the production of messenger RNA by the process of transcription. A promoter is "operatively linked" to a gene when the initiation of the transcription process at the promoter leads to the production of messenger RNA encoded by that gene.

The term "signal sequence," as used herein, refers to a DNA sequence that encodes an amino acid sequence that signals the host cell to perform a specific task with the resulting protein. For example, a signal sequence may instruct a host cell to secrete the encoded protein rather than to keep it inside the cell. The term "termination sequence," as used herein, means a DNA sequence that stops the process of transcription. A terminator sequence normally follows the DNA sequence of the gene of interest in a plasmid.

Another aspect involves transforming a host cell with the recombinant DNA encoding lysostaphin. The term "host cell," as used herein means any cell, prokaryotic or eukaryotic, including animal and plant cells, that may be transformed or transfected with the recombinant DNA of the invention. The host cell may be a bacterium, for example, *Eschericia. coli,* Lactococcus *lactis, Bacillus sphaericus,* and related organisms. Genetic elements in the recombinant DNA described herein such as the origin of replication, the promoter, the signal sequence, and the termination sequence are often host cell specific. Thus additional molecules include recombinant DNA molecules that contain elements for these functions that work in the specific host cell used.

The term "transform," as used herein, means the introduction of a DNA molecule into a bacterial cell. Bacterial cells are made "competent" when they will readily receive foreign DNA molecules. Methods for making bacterial cells competent and for transforming these competent cells are standard and known to those of skill in the art. Bacteria may also be transformed by electroporation. The term "transfect," as used herein, means the introduction of a DNA molecule into a mammalian host cell. A mammalian host cell may be transfected by several common methods including electroporation, calcium phosphate precipitation, DEAE-Dextran, and liposome reagents (i.e., Lipofectin).

The production of active homogenous lysostaphin and active truncated homogenous lysostaphin from host cells that are transformed or transfected with a recombinant DNA molecule expressing lysostaphin is also described herein. The term "homogenous lysostaphin," as used herein, means a preparation of lysostaphin in which all of the lysostaphin molecules begin at the same amino acid in the lysostaphin protein sequence. Recombinant lysostaphin produced in bacteria may have residual, uncleaved F-Met on the N-termini of some molecules and recent studies show that 20% and 2% of recombinant molecules produced in in *L. lactis* and *E. coli,* respectively, contain residual F-Met moieties. Consequently, a recombinant homogeneous lyphostatin may include bacterially-produced lysostaphin or truncated lysostaphin with and without N-terminal F-Met residues. In some embodiments, greater than 50%, 60%, 70%, 80%, or 90% of the recombinant molecules do not have residual F-Met moieties.

The term "heterogeneous lysostaphin," as used herein, means a preparation of lysostaphin that contains a mixture of lysostaphin molecules that begin at different amino acids in the lysostaphin sequence. The active homogenous lysostaphin may be either full length lysostaphin or truncated lysostaphin.

A lysostaphin protein is "active" when it exhibits proteolytic activity, *in vitro* and *in vivo,* to cleave the specific cross-linking polyglycine bridges in the cell walls of staphylococci. A lysostaphin protein has "enhanced staphylolytic activity" when it exhibits increased proteolytic activity against polyglycine bridges in the cell walls for staphylococci as compared to full length lysostaphin. When lysostaphin is expressed inside the host cell, the host cell may be lysed and lysostaphin isolated from the resulting cell extract. When lysostaphin is secreted into the host cell media, lysostaphin may be concentrated and then purified using standard techniques such as column chromatography, hydrophobic purification, and ion-exchange chromatography. For purposes of large scale production, host cells may be grown in, for example, large roller bottles or large volume fermenters.

Homogenous, recombinant lysostaphin may be added to a variety of carriers. Such vehicles increase the half-life of the lysostaphin in the nares following instillation into the nares. These carriers comprise natural polymers, semi-synthetic polymers, synthetic polymers, liposomes, and semi-solid dosage forms (10, 11, 12, 14, 15, 18, 20, 21). Natural polymers include, for example, proteins and polysaccharides. Semi-synthetic polymers are modified natural polymers such as chitosan, which is the deacetylated form of the natural polysaccharide, chitin. Synthetic polymers include, for example, dedrimers, polyphosphoesters, polyethylene glycol, poly (lactic acid), polystyrene sulfonate, and poly (lactide coglycolide). Semi-solid dosage forms include, for example, creams, ointments, gels, and lotions. These carriers can also be used to microencapsulate lysostaphin molecules or can be covalently linked to lysostaphin.

The resulting lysostaphin compounds may be administered to a patient at risk for staphylococcal infection by several routes. Patients at risk, include health care workers, newborns and premature infants, persons undergoing inpatient or outpatient surgery, burn victims, patients receiving indwelling catheters, stents, joint replacements and the like, geriatric patients, and those with genetically, chemically or virally suppressed immune systems. Among non-human patients, those at risk include zoo animals, herd animals, and animals maintained in dose quarters.

Representative patients include any mammal subject to *S. aureus,* or other staphylococcal infection or carriage, including humans and non-human animals such as mice, rats, rabbits, dogs, cats, pigs, sheep, goats, horses, primates, ruminants including beef and milk cattle, buffalo, camels, as well as fur-bearing animals, herd animals, laboratory, zoo, and farm animals, kenneled and stabled animals, domestic pets, and veterinary animals.

A lysostaphin composition may be instilled into the nares of a patient. Alternatly, a lysostaphin composition may be rubbed onto the skin, applied to an open wound, or injected for systemic administration. In addition, these lysostaphin compositions may be administered in conjunction with antibiotic anti-staphylococcal drugs including antibiotics like mupirocin and bacitracin; anti-staphylococcal agents including lysozyme, mutanolysin, and cellozyl muramidase; anti-bacterial peptides like nisin; and lantibiotics, or any other lanthione -containing molecule, such as subtilin. The compositions of the invention may also include anti-staphylococcal agents including anti-staphylococcal monoclonal antibodies, for example antibodies directed against peptidoglycan (PepG), described in U.S. Provisional Application 60/343,444, filed December 21, 2001, and *Multifunctional Monoclonal Antibodies Directed To Peptidoglycan of Gram-Positive Bacteria,* filed herewith.

The recombinant lysostaphins and pharmaceutical compositions of the invention may be administered by intravenous, intraperitoneal, intracorporeal injection, intra-articular, intraventricular, intrathecal, intramuscular or subcutaneous injection, or intranasally, dermally, intradermally, intravaginally, orally, or by any other effective method of administration. The composition may also be given locally, such as by injection to the particular area infected, either intramuscularly or subcutaneously. Administration can comprise administering a pharmaceutical composition by swabbing, immersing, soaking, or wiping directly to a patient. The treatment can also be applied to objects to be placed within a patient, such as dwelling catheters, cardiac valves, cerebrospinal fluid shunts, joint prostheses, other implants into the body, or any other objects, instruments, or appliances at risk of becoming infected with a staphylococcal bacteria, or at risk of introducing such an infection into a patient.

The present invention is further illustrated by the following examples that teach those of ordinary skill in the art how to practice the invention. The following examples are merely illustrative of the invention and disclose various beneficial properties of certain embodiments of the invention. The following examples should not be construed as limiting the invention as claimed.

## EXAMPLES

### Example 1

### Construction of an Expression Plasmid for Intracellular Expression of Homogenous Truncated Lysostaphin

Polymerase Chain Reaction (PCR) was used to amplify a fragment of the lysostaphin gene and the resulting fragment was cloned into pBAD/gIII expression vector (Invitrogen). A form of truncated lysostaphin was generated and fused directly to a translation start methionine for intracellular expression in *E. coli*. Figure 1 provides the cloning strategies used for production of the plasmid of this example and Example 2 below.

To complete the fusion of the lysostaphin expression cassettes, the 5' and 3' ends were amplified from different templates and then connected using overlap extension PCR ("OLE-PCR"). Specifically, the 5' fragment containing the upstream expression components was amplified from the pBAD/gIII plasmid and the 3' end, containing the lysostaphin coding sequence, was amplified from a sample of host cells containing the lysostaphin gene, provided by Ambi, Inc. The PCR amplification reactions for the 5' fragment contained 10 ng of template DNA, 20 pmoles of primers JSBX-29 and JSBX-53 (see Table 1), 2.5 units of *ExTaq* polymerase (PanVera), 1 x *ExTaq* reaction buffer, 200 μM dNTP, 2 mM $MgCl_2$ in a 50 μl reaction volume. The template was denatured by an initial incubation at 96°C for 3 min. The products were amplified by 25 thermal cycles of 96°C for 30 sec., 56°C for 30 sec., 72°C for 30 seconds. The PCR amplification reactions for the 3' fragment contained 10 ng of template DNA, 20 pmoles of primers JSBX-34 and JSBX-52 (see Table 1), 2.5 units of *ExTaq* polymerase, 1x *ExTaq* reaction buffer, 200 μM dNTP, 2mM $MgCl_2$ in a 50 μl reaction volume. The template was denatured by an initial incubation at 96°C for 3 min. The products were amplified by 25 thermal cycles 96°C for 30 sec., 52°C for 30 sec., 72°C for 30 seconds. The PCR products from the successful reactions were purified using the Nucleospin PCR Purification system (Clontech) per the manufacturer's procedure.

Table 1

| Primer | Primer Sequence (5' to 3') | SEQ ID NO: |
|---|---|---|
| JSBX-29 | ATTAGCGGATCCTACCTGAC | 1 |
| JSBX-32 | CCATTGTGCTGAATGTTCATGTGTGCTATGGCTATAGAACGGC | 2 |
| JSBX-33 | GGTGCCGTTCTATAGCCATAGCACACATGAACATTCAGCACAATGG | 3 |
| JSBX-34 | TTATTCTTCTAGATCACTTTATAGTTCCCCAAAGAAC | 4 |
| JSBX-35 | CTATGCCATAGCATTTTTATCC | 5 |
| JSBX-36 | AGCCAAGCTGGAGACCG | 6 |
| JSBX-52 | GGGCTAACAGGAGGAAGCTTCCATGACACATGAACATTCAGCAC | 7 |
| JSBX-53 | TGTGTCATGGAAGCTTCCTCCTGTTAGCCC | 8 |

OLE- PCR was then performed using 2 μL of the purified 5' fragment, 0.5 μL of the purified 3' fragment, 20 pmol of primers JSBX-29 and JSBX-34, 2.5 units of *ExTaq* polymerase, 1x *ExTaq* reaction buffer, and 200 μM dNTP, 2mM $MgCl_2$ in a 50 μl reaction volume. The template was denatured by an initial incubation at 96°C for 3 min. The products were amplified by 30 thermal cycles 96°C for 30 sec., 56°C for 30 sec., 72°C for 30 seconds. The first 5 cycles were performed without the addition of primer DNA to optimize the likelihood that the 5' and 3' sections, when denatured, would overlap appropriately to allow amplification of the entire lysostaphin sequence and accessory elements (i.e., signal sequence). The PCR products from successful reactions were purified using the Nucleospin PCR Purification system per manufacturer's instructions.

The PCR products, approximately 1000 base pairs in length, were then digested with restriction endonucleases *Bam*HI and *XbaI,* and cloned into a bacterial vector, pBAD/gIII, for protein expression. The digested PCR fragments were ligated into de-phosphorylated, *Bam*HI and *XbaI* digested pBAD/gIII, using ligase (Promega) and following the manufacturer's instructions using a 3:1 insert to vector molar ratio. One half (5 μl) of the ligation reactions were used to transform competent TOP10 cells (Invitrogen) per the manufacturer's instructions. Bacterial clones containing plasmids with DNA inserts were identified using diagnostic restriction enzyme digestions with *Pfl*MI (New England Biolabs). The expected pattern of banding in this diagnostic digest was one band at 4000 bp and one band at 800 bp for pJSB20 and 4000 bp and 750 bp for pJSB28. DNA sequencing was performed using cycle sequencing reactions primed by JSBX-35 (SEQ ID NO: 5) and JSBX-36 (SEQ ID NO: 6) and analyzed on a CEQ2000 capillary sequencer (Beckman/ Coulter). Sequencing was carried out by mixing 500 ng of plasmid preparation, 10 ng of sequencing primer, and 8 μl of CEQ2000

sequencing mix (Beckman Coulter PN 608000). The sequencing samples were incubated at 96°C for 2 minutes and then amplified by 96°C 20 seconds, 50°C for 20 seconds, and 60°C for 4 minutes for a total of 40 cycles.

The resulting plasmid, pJSB28, is shown in Figure 2. This figure also shows the sequence of the truncated lysostaphin gene that is present in this plasmid (SEQ ID NO: 9 for amino acid sequence and SEQ ID NO: 10 for nucleotide sequence).

## Example 2

### Construction of an Expression Plasmid for Extracellular Expression of Truncated Lysostaphin

As described above, PCR was used to amplify a fragment of the lysostaphin gene and the resulting fragment was cloned into pBAD/gIII expression vector (Invitrogen). In this example, a form of truncated lysostaphin was generated and fused to a signal sequence for secretion into the periplasmic space of *E. coli.* OLE-PCR was also used to complete the fusion of the lysostaphin expression cassettes as described above, except that the 5' fragment contained the upstream expression components and the signal sequence for extracellular expression. These 5' components were also amplified from the pBAD/gIII plasmid. The PCR amplification reactions for the 5' fragment were as described above except primers JSBX-29 and JSBX-32 were used. Likewise, the 3' fragment was amplified as described above except that primers JSBX-34 and JSBX-33 were used. OLE-PCR, the cloning of the truncated lysostaphin insert into the pBAD/gIII vector, the diagnostic digest, and subsequent sequencing were all performed as described above.

The resulting plasmid, pJSB20, is shown in Figure 3. This figure also shows the sequence of the truncated lysostaphin gene and signal sequence that is present in this plasmid (SEQ ID NO: 11 for amino acid sequence and SEQ ID NO: 12 for nucleotide sequence).

## Example 3

### Small Scale Production of Active Truncated Lysostaphin in *E.coli*

Overnight cultures of TOP10 cells transformed with pJSB20 or pJSB28 grown at 37°C were diluted 1:100 in 200 ml LB media supplemented with 100 μg/mL ampicillin. When the $OD_{600}$ reached 0.5, arabinose was added to 0.2% to induce expression of the lysostaphin protein. The cultures were then allowed to grow for 48 hours at 30°C. Cells were pelleted by centrifugation at 5000 X g for 15 minutes. In the case of the intracellular lysostaphin (pJSB28), the cell pellet was treated with B-Per extraction reagent (Pierce) supplemented with 0.125M NaCl following the manufacturer's instructions and the supernatant was collected and analyzed for the presence of lysostaphin and whether the enzyme was active. In the case of secreted lysostaphin (pJSB20), the media supernatant was collected, concentrated using an Amicon spiral ultrafiltration concentrator (S1Y-10 with a 10 KD cutoff) and analyzed for the presence of lysostaphin and for lysostaphin activity.

To determine if lysostaphin was expressed either intracellularly or by secretion into the host cell media, the presence of lysostaphin in the resulting sample was analyzed by ELISA. Polyclonal anti-lysostaphin was generated in rabbits by primary vaccination with 100 μg of lysostaphin to the popliteal lymph nodes. The animals were given 100 μg booster injections once per month for the following three months before being bled to produce the polyclonal serum. In the ELISA, 100 μl of the resulting rabbit polyclonal anti-lysostaphin serum was diluted 1:10,000 in PBS and used to coat wells of a 96-well microtiter plate (NUNC, Macrosorb) overnight at 4°C. The plates were then washed with PBS and blocked with 100 μl/well of 1% BSA in PBS at room temperature for 30-60 minutes. Experimental samples and the lysostaphin standard (Ambicin L, AMBI, Inc.) were diluted in PBS with 0.1 % Tween and 0.1 % BSA (PBS-T-BSA). The anti-lysostaphin coated, blocked plates were then washed with PBS-T four times. The samples and standard dilutions were then transferred (100μl/well) onto an anti-lysostaphin coated plate and incubated for 30-60 minutes at room temperature. The plate was then washed 4 times with PBS-T. The coating polyclonal antibody was also used as the detection antibody by biotinylating the antibody with Biotin-sulfo-N-hydroxysuccinimide caprylate (Bioaffinity Systems, Inc.) according to the manufacturer's instructions. This biotinylated antibody preparation was then diluted 1:800 in PBS-T-BSA and added at 100 μL/well. The plate was incubated 30-60 minutes at room temperature and then washed 4 times with PBS-T. ExtraAvidin-HRP (Sigma Cat# E2886) was diluted 1:8000 in PBS-T-BSA and then 100μL/well was added to the plate which was incubated for 30-60 minutes. The plate was washed 4 times with PBS-T. One hundred microliters per well of TMB-Microwell Substrate (BioFx Cat# TMBW 0100-01) was added and the reaction was allowed to proceed for 3-5 minutes before being stopped by the addition of TMB Stop reagent (BioFx Cat# STPR 0100-01). Absorbance was then read at 450 nm. As shown in Figure 4, both the pJSB28 and pJSB20 DNA plasmids expressed lysostaphin when transformed into *E. coli* host cells.

To determine if the lysostaphin expressed by pJSB28 and pJSB20 was active, a lysis assay was used. In this assay, *S. aureus* bacteria were heat killed and then incubated with varying concentrations of the lysostaphin preparation.

Lysostaphin activity was detected when the experimental preparation caused the bacterial suspension to lose its turbidity and become clear. Specifically, the lysostaphin positive control (Ambicin L) and samples were diluted to concentrations of 200, 100, 50 and 10 μg/ml in 50 μl of PBS. *S. aureus 5* was heat killed by incubation at 56°C for 3 hours. These heat killed bacteria were suspended to approximately $OD_{650}$ 0.9-1.0 in PBS. Then, 450 μl of this suspension was placed in a disposable cuvette and the $OD_{650}$ was determined. This was the 0 time point. Fifty microliters of the sample or control was then added, bringing the final concentration of lysostaphin in the samples to 20, 10, 5 and 1 μg/ml. The $OD_{650}$ of the suspension was thereafter measured every 60 seconds for 10 minutes. Figure 5 is a typical kinetic curve for the 10 μg/mL sample of lysostaphin expressed by pSJB28 and pSJB20. PBS alone was used as a negative control in the assay. In this assay, the $OD_{650}$ never reaches zero due to the presence of bacterial debris that remains even after complete lysis of the sample.

**Example 4**

**Truncated Lysostaphin has Enhanced Staphylolytic Activity Over Full Length Lysostaphin**

The staphylolytic activity of the truncated "THE" form of lysostaphin was compared to the staphylolytic activity of the full length "AATHE" form of lysostaphin using a fluorescence emission assay as described in Kline et al. (6). In the current assay, fluorescamine was substituted for TNBS to detect amines revealed when lysostaphin cuts the N-acetyl hexaglycine substrate.

Lysostaphin samples were prepared by first determining the optical density of the enzyme using a Spectrophotometer at OD 280 nm ($OD_{280}$). The spectrophotometer was zeroed with lysostaphin dilution buffer. 1:10 dilutions were prepared by adding 100 μl of enzyme solution to 900 μl lysostaphin dilution buffer (20mM Sodium Acetate, and 0.5% Tween 20, pH 4.5) and vortex mixing. The samples were then read at $OD_{280}$. The concentration (mg/ml) of lysostaphin in the enzyme sample was calculated by multiplying the resulting $OD_{280}$ values by the dilution factor of 10 and by the extinction coefficient of 0.41. For use in this assay, each lysostaphin sample was then diluted to 40 μg/ml, using lysostaphin dilution buffer.

The hexaglycine substrate was prepared by as described by Kline et al. (6). Specifically, a 10 mM stock was prepared by resuspending 40.2 mg N-acetylhexaglycine (N-Ac-Hex; FW=402) in 1 ml of water, adding sodium hydroxide until the solid dissolved in solution. The final volume of the N-Ac-Hex stock was adjusted to 10 mls by adding substrate/assay buffer (5mM Trisodium citrate, 1 mM Disodium EDTA, 0.1M Sodium Borate, pH 8.0). An aliquot of this stock was further diluted 1:10 with substrate/assay buffer before use in the assay.

Each lysostaphin sample was transferred to a microwell plate and diluted 1:3 (starting from 40 μg/ml) in dilution buffer. After dilutions were complete, the final volume in each well was 60 μl. Blank wells lacking each component were also prepared. Each sample was done in triplicate. Once the lysostaphin sample dilutions were prepared, 60 μl of N-Ac-Hex substrate was added to each well.

The assay plates were then incubated at 37°C for 1 hour. 40 μl of 0.3 mg/ml fluorescamine (Molecular Probes) in acetone was then added to each well and the sample read immediately. Samples were read using a fluorescent plate reader (Molecular Devices) which was set at 390 nm excitation, 475 nm emittence, and 420 nm cut off. Negative controls consisted of the matching well(s) containing lysostaphin, buffers, and fluorescamine, but lacking N-Ac-hexaglycine. The fluorescence of wells containing the corresponding concentration of lysostaphin but lacking the N-Ac-Hex substrate were subtracted from each reading. Samples were done in triplicate, the values averaged, and the standard deviation calculated. The results of this experiment are shown in Figure 6A.

A time course experiment was also performed using a uniform concentration of lysostaphin in each experimental well. As described above, negative controls consisted of the matching well(s) containing lysostaphin, buffers, and fluorescamine, but lacking N-Ac-hexaglycine. Experimental wells contained lysostaphin at a final concentration of 20 μg/ml. Samples were incubated at 37°C for 5 minutes, 20 minutes, 40 minutes, 60 minutes, and 90 minutes. When the incubation period lapsed, 40 μl of 0.3 mg/ml fluorescamine in acetone was added to each well and the sample read immediately. Samples were read and analyzed as described above. The results of this experiment are shown in Figure 6B.

Figures 6A and 6B demonstrate that the truncated "THE" form of lysostaphin was more active than the full length "AATHE" form. The colorimetric assay monitors the action of each lysostaphin molecule on a substrate molecule whereas the *S. aureus* viability assay simply measures whether there was enough lysostaphin activity to kill a *S. aureus* bacterium. Figures 6A and 6B show that truncated lysostaphin has increased staphylolytic activity over the full length "AATHE" form. In turn, lysostaphin compositions produced with this truncated lysostaphin would provide enhanced staphylolytic activity over compositions made with heterologous lysostaphin or homologous full length lysostaphin.

**Example 5**

**Large scale purification of Active Truncated Lystostaphin in L. lactis Cloning of the lysostaphin gene:**

The entire lysostaphin gene was amplified from the plasmid pRN5550 using the polymerase chain reaction as generally described above (PCR). pRN5550 had previously been used for lystostaphin production at AMBI Inc., Purchase, NY. The resulting amplified PCR product contained the coding region for the production of lysostaphin, a 5' TTG codon for translation initiation, and a 3' *XbaI* restriction site for cloning purposes. This PCR product was then digested with *XbaI* and ligated into a standard *L. lactis* expression plasmid, pNZ8148ScaI, which had been cut with ScaI and *XbaI.* This ligation created the expression plasmid pLss1C.

Sequence analysis confirmed that the lysostaphin coding sequence in pLss1C was correct. This included a known silent mutation at position 256 (T to C; Asn to Asn) present in the lystostaphin coding sequence in pRN5550. (SEQ ID NO: 13 for amino acid sequence and SEQ ID NO: 14 for nucleotide sequence) The truncated lysostaphin (the mature lysostaphin coding sequence lacking the two 5'-most Ala codons of the wild-type mature protein) was then amplified by PCR using pLss1C as a template. The resulting amplified product contained the coding region for truncated lysostaphin, a 5' TG sequence for translation initiation (together with the last T of the cut restriction site *ScaI*, a TTG codon is constituted that can serve as an initiation codon in Lactococci), and a 3' *XbaI* restriction site for cloning purposes. This PCR product was then digested with *XbaI* and ligated into pNZ8148ScaI cut with *ScaI* and *XbaI,* resulting in plasmid pLss12C (not shown). Finally, the chloramphenicol resistance gene was exchanged for the lacF gene (originating from plasmid pNZ8148F-1, not shown) using the restriction sites *SalI* and *NspV,* resulting in plasmid pLss12F. Restriction maps of pRN5550, pNZ8148ScaI, pLss1C, and pLss12F can be found respectively in Figures 7A-D.

In pLss12F, the theoretical N-terminal amino acid sequence for truncated lysostaphin is f-Met-Thr-His-Glu. In many cases, the f-Met is cleaved off by a dedicated peptidase. N-terminal amino acid sequencing results to date suggest that indeed this 5' f-Met is sometimes cleaved off.

pLss12F was isolated and purified by standard methods as described in Sambrook, J., *et al.* (17).

**Transformation of host cells with the expression plasmid:**

L. lactis subsp. *cremoris* NZ3900, a derivative of strain MG1363 *(pepN:: nisRK, lac*$^+$ *lacF,* prophage-cured), was transformed with pLss12F and plated on Elliker agar supplemented with 1% lactose. Transformants were selected on the basis of their ability to grow on this agar and to ferment lactose. After screening for correct plasmid retention, the resulting strain was called NZ3900 (pLss12F).

**Initiation of Seed Train in Laboratory:**

Each of two vials of NZ3900 (pLss12F) Initial Cell Bank Working Stock were inoculated into 300 mL of Soy Peptone/Yeast Extract Medium and incubated overnight (16 to 24 hours) at 30 $\pm$ 3°C.

**Seed Train - 30 L Fermentation:**

The overnight cultures were used to inoculate 30 L of Soy Peptone/Yeast Extract Medium in a Chemap 75 fermentor. The fermentor ran overnight (16 to 20 hours) at 30 $\pm$ 3°C.

**Production - 3000 L Fermentation:**

Three thousand liters of Soy Peptone/Yeast Extract Medium in a 4000 liter fermentor were inoculated with the 30 liter seed culture. Inclusion of approximately 3.4 $\mu$m zinc in the fermentation media may maintain or increase lysostaphin activity, since zinc is required for lysostaphin activity. The fermentation proceeded at 30 $\pm$ 2°C and pH 6.5 $\pm$ 0.2; pH was regulated with 5 M NaOH. Induction using Nisin at a concentration of 10 ng/mL was performed when the culture reached an $OD_{600}$ of approximately 1.0. Lysostaphin production proceeded for 6 hours before the process was stopped by cooling the fermentor content down to below 10°C.

**Concentration of Biomass:**

The entire *L. lactis* biomass of the fermentation was concentrated on a 3.8 $m^2$ 0.8 $\mu$m membrane by microfiltration. The cells were then diafiltered (200%) against water.

**Release of Lysostaphin - Homogenization:**

The concentrated cells were homogenized 3 times in 150 mM NaCl/25 mM Na phosphate, pH 7 buffer in an APV homogeniser at < 10°C, releasing lysostaphin into the homogenate.

**Removal of Cellular Debris:**

Cellular debris was removed from the liquid homogenate by ultrafiltration using a 500-750 kDa ultrafiltration membrane. Following ultrafiltration, the lysate was in approximately 150 mM NaCl, 25 mM sodium phosphate pH 7, and was diluted 1:2 with water. Dilution reduces the solution conductivity below 10 mS/cm to allow efficient lysostaphin capture on the cationic chromatography resin.

**Capture - SP-Sepharose Chromatography:**

The permeate was adjusted to pH 7.2 with 0.4 M $Na_2HPO_4$, filtered on a Sartobran P filter capsule, and purified on SP-sepharose in a BPG3000 column that had been equilibrated with two column volumes of 50 mM Sodium Phosphate, pH 7.5. After washing with three column volumes of this buffer, the lysostaphin was eluted with two column volumes of 25 mM Sodium Phosphate, 0.25 M NaCl, pH 7.5. All eluted fractions containing lysostaphin were collected and stored at <-20°C.

**Dilution/Filtration:**

Approximately twelve liters of SP-Eluate was removed from the freezer and allowed to thaw at room temperature for approximately 18 hours. The material was pooled in a single container and was then mixed gently to create a homogeneous solution. Twelve liters of Q Equilibration Buffer (0.05 M Tris, pH 7) was added to the SP-Eluate pool to bring the conductivity of the solution to the target range of 8-16 mS/cm. The mixture was run through a 0.45 μm Sartoclean filter unit into a 50 liter Stedim bag to create the Filtered SP-Eluate Pool.

**Q-Sepharose Purification:**

The Filtered SP-Eluate Pool was purified on a previously qualified Q-Sepharose FF Chromatography column that had been regenerated with approximately two column volumes of Q Regeneration Buffer (0.05 M Tris, 0.04 M NaOH, 1 N NaCl) and was subsequently equilibrated with approximately three column volumes of Q Equilibration Buffer (0.05 M Tris, 0.04 M NaOH). The Filtered SP-Eluate Pool was loaded onto the column until 6 liters of Q Flow Through (Q F-T 1) had been collected in a waste container. The remaining pooled material was loaded onto the column and the remaining flow through (Q F-T 2) was collected in a lined tank. Approximately twelve liters of Q Equilibration Buffer was then run onto the column. A total of 29.38 liters of Q F-T 2 material was collected.

**Q Flow Through - Dilution/Filtration:**

Ammonium Sulfate (3M $(NH_4)_2SO_4$) was mixed into the solution [volume of ammonium sulfate added = (Q F-T 2) ÷ 2.75] over the course of 15 minutes; the pH of the solution was then adjusted to 6 - 7 using Phosphoric Acid. The Q F-T 2 was filtered (0.45 μM) into a single bag and stored overnight at 2 - 8°C (Filtered Q Eluate).

**Phenyl Sepharose Purification:**

The following day, the Filtered Q Eluate was purified on a previously qualified Phenyl-Sepharose HP column that had been equilibrated with approximately two column volumes of Phenyl Equilibration Buffer (0.05 M $NaH_2PO_4$, 0.8 M $(NH_4)_2SO_4$,).

After loading the Filtered Q Eluate on the column, it was rinsed with approximately two column volumes of Phenyl Rinse Buffer (0.05 M $NaH_2PO_4$, 0.5 M $(NH_4)_2SO_4$). Purified lysostaphin API was eluted with Phenyl Elution Buffer (0.05 M $NaH_2PO_4$, 0.25 M $(NH_4)_2SO_4$). Approximately 51 liters of Phenyl Eluate was collected ($OD_{280}$ of the eluate was > 0.2). The material was stored at ambient temperature overnight.

**Ultrafiltration/Diafiltration:**

The following day, a sanitized Millipore Pellicon Ultrafiltration Assembly equipped with a 20 $ft^2$ 5 KD molecular weight cut off (MWCO) Pellicon Cassette was equilibrated with 3 liters of Phenyl Regeneration Buffer and drained. The Phenyl Eluate was concentrated by ultrafiltration until a target lysostaphin concentration (as determined by $OD_{280}$ measurement) of approximately 25 mg/mL had been achieved. The concentrated Phenyl Eluate was then diafiltered against WFI. The permeate was collected in a lined tank until the conductance of the material was reduced to 0.5 - 0.6 mS/cm. The protein concentration of the Diafiltered Concentrate was determined by $OD_{280}$ and additional permeate was collected to obtain a target lysostaphin concentration of approximately 25 mg/mL. The Pellicon apparatus was rinsed and drained and the rinse volume was added to the collected permeate (Diafiltered Phenyl Eluate, Formulated Concentrate). Filtration:

The Formulated Concentrate was filtered through a Millipak 200 unit (0.2 μM filter) into two separate Stedim 5 liter bags (Bag 1 and Bag 2) to create Bulk API. Samples were collected from each bag and were submitted for analytical, bioburden, and endotoxin testing.

**Bulk API (Active Pharmaceutical Ingredient):**

The Bulk API, Diafiltered Q- and Phenyl Sepharose purified lysostaphin was stored at 2 - 8°C. Figure 9 shows an example of lysostaphin API on an SDS-PAGE reducing gel.

## Example 6

### Comparison Of Homogenous Mature and Homogenous Truncated Lysostaphin By Killing Assay With Different Concentrations of Lysostaphin

Homogenous mature (AATHE) and truncated lysostaphins (THE), prepared essentially as described above, were compared in a standard potency assay as follows:

**Preparation of lysostaphin working stocks for assay:**

Approximately 1 milligram of lysostaphin powders were dissolved in 1ml each of PBS to create stock samples. Stock samples were diluted 1:5 and 1:10 (200μl in 800μl and 100μl in 900μl). The $A_{280}$ of the 1:5 and 1:10 dilutions was read on a BioRad SmartSpec 3000 spectrophotometer blanked with PBS. Concentrations (in mg/ml) of the stock solutions were determined by multiplying the absorbance of each dilution by 0.49 (the reciprocal of the extinction coefficient) and the dilution factor (5 or 10) to determine the concentration in mg/ml. Stock solutions were only used where the determined concentrations for each dilution was within 10% of each other. Stock solutions were further diluted to 5 to 6 ng/μl working stocks with PBS and kept on ice until used. Aliquots of working stocks were stored at -70°C.

**Bacterial Preparation:**

Frozen stocks of *S. aureus* type 5 ATCC 49521 were streaked on blood agar and incubated overnight. Three colonies were transferred to 1 ml of DIFCO tyrptic soy broth and incubated overnight at 37°C w/ shaking to form a bacterial stock. The bacterial stock was normalized with PBS to an $OD_{650}$ of 0.750 to 0.790. 1:10 serial dilutions of the normalized stock were made in PBS. Actual starting CFUs were determined by mixing 350 ul of normalized stock with 650μl of PBS and plating 100μl on blood agar.

**Lysostaphin Dilutions:**

Three independent sets of dilutions were made for each lysostaphin (18 tubes per sample, 6 tubes per set, 3 tubes per series). Each set contained two dilution series of 48, 24, 12 ng/ml lysostaphin and 32, 16, 8 ng/ml lysostaphin. This resulted in 18 tubes for each lysostaphin with 650μl per tube for each sample divided into 3 sets per sample (each set consists of 48, 32, 24, 16, 12, and 8 ng).

**Performance of assay:**

To start the reaction, 350μl of working stock bacteria was added to each of the 18 tubes in the sample set and mixed by vortexing. The samples were incubated for 20 minutes at room termperature with additional mixing at 10 minutes and just prior to plating. After 20 minutes, 100μl samples from each tube are plated onto blood agar plates and incubated overnight at 37°C. Colonies were enumerated and the results plotted. The results of three such assays, depicted in Figures 10-12, demonstrate the generally greater bacteriocidal activity of the truncated lyphostaphin (THE ...) as compared to the mature form (AATHE...) over a wide concentration range.

**Example 7**

**Comparison Of Homogenous Mature and Homogenous Truncated Lysostaphin By Time Course Killing Assay On Cultures Grown in Whole Blood**

Lysostaphin is generally less active in whole blood than in a buffer. To further examine the enhanced activity of truncated lysostaphin versus full length lysostaphin, homogenous mature and truncated lysostaphins were compared in a time course killing assay in whole blood as follows. Cultures of S. aureus were grown overnight in DIFCO tryptic soy broth. 50μl aliquots of the overnight S. aureus cultures were used to inoculate six tubes containing 1 ml of fresh human blood each. Inoculated bloods were grown overnight at 37°C with shaking and pooled the next day. 1 ml aliquots of the pooled blood cultures were mixed with 1 microgram of homogeneous mature (AATHE) or truncated (THE) lysostaphin and incubated at room temperature. At specified time points, 100μl samples of each were diluted 1:100 in PBS. 100 μl of each PBS dilution was plated on a blood agar plate and cultured overnight at 37°C to allow colony growth. Colonies were enumerated and the results plotted in Figures 13 and 14. These results indicate that the greater bacteriocidal activity of truncated lyphostaphin (THE. ..) as compared with the mature form (AATHE...) appears more pronounced at longer incubation times.

**Example 8**

**Comparison Of Homogenous Mature and Homogenous Truncated**

**Lysostaphin By Optical Density Drop Assay**

Homogenous mature and truncated lysostaphins were compared in a time course turbidity drop assay as follows.

**Test sample preparation:**

Lysostaphin stock solutions were prepared in PBS as above and diluted in PBS to approximately 0.1 mg/ml working stocks.

**Preparation of bacteria for assay:**

3-5 ml cultures of Staphylococcus carnosus were grown overnight in DIFCO tryptic soy broth from a blood agar plate <1 week-old. 1.5 milliliters of overnight S. carnosus culture was pelleted by microfuged at maximum rpm for 5 minutes. The bacterial pellet was washed with an equal volume of PBS and repelleted. The washed pellet was resuspended in 3 ml PBS diluted with PBS to a final OD650 of 1.55+/-0.04. The diluted bacteria were stored on ice until use.

**Performance of assay:**

The drop in turbidity of the resuspended *S. carnosus* was followed on a BioRad SmartSpec 3000 blanked against PBS and set for "Kinetics" assay, using as parameters; 650nM, 900 sec total duration, 30 sec interval, and no background subtraction. For each lysostaphin preparation, 500ul of the -1.55 absorbance bacteria were pipetted into a fresh cuvette and allowed to warm to room temperature.. A zero reading was determined, followed rapidly by the addition and mixing of 1ug (10ul) of lysostaphin (either AATHE or THE). The OD650 of each mixture was determined over a period of sixty minutes and the lysostaphin activity determined according to the following formula:

$$\frac{\text{(Time (min) to } OD_{650} \text{ of reference lysostaphin *ug added)}}{\text{(Time (min) to } OD_{650} \text{ of sample lysostaphin *ug added)}} * 100 = \text{Units/ug}$$

The results of two optical density drop assays, shown in Figures 15 and 16, illustrate the more rapid bacteriocidal activity of the truncated lyphostaphin (THE ...) than the mature form (AATHE...). The inventors note that this assay was terminated at 60 minutes. In vivo, where incubation times are measured in hours and days, activity of truncated lysostaphin may be even more markedly superior than that of the mature form.

## CONCLUSION

Thus, Examples 1 and 2 describe the design and production of recombinant DNA molecules that express a homogenous form of lysostaphin. These plasmids allow for either intracellular expression of lysostaphin or for secretion of lysostaphin extracellularly. Example 3 shows that these plasmids, when transformed into bacterial host cells, do express lysostaphin and that this lysostaphin is enzymatically active. Example 4 demonstrates that the truncated form of lysostaphin exhibited greater antistaphylococcal activity than its "mature" full length counterpart. Example 5 describes a bulk purification protocol for recombinant lysostaphin. Examples 6,7, and 8 demonstrate that the truncated THE form of lysostaphin has superior anti-staphylococcal activity over the mature AATHE form of lysostaphin.

One of skill in the art would realize that the recombinant DNA molecules described herein are not limited to only those described in the above examples. One of ordinary skill would know that alternate host cells may be used for expression. Such a person would also know to substitute genetic elements on the recombinant DNA molecule, such as promoters and replication origins, for alternate sequences that achieve the same function in that alternate host cell. These alternate sequences are well known to those of ordinary skill in the art. Further, the instant invention is not limited to lysostatphin that is purified by the above-described methods. One of ordinary skill in the art would be able to use alternate methods of protein purification to achieve the same ends of the invention.

References:


1. Chang, F. Y., N. Singh, T. Gayowski, S. D. Drenning, M. M. Wagener and I. R. Marino. 1998. Staphylococcus aureus nasal colonization and association with infections in liver transplant recipients. Transplantation 65:1169-1172.
2. Chapoutot, C., G.-P. Pageaux, P. F. Perrigault, Z. Joomaye, P. Pemey, H. Jean-Pierre, O. Jouquet, P. Blanc and D. Larrey. 1999. Staphylococcus aureus nasal carriage in 104 cirrhotic and control patients A prospective study. J. Hepatol. 30:249-253.
3. Fierobe, L., D. Decre, C. Muller, J.-C. Lucet, J.-P. Marmuse, J. Mantz and J.-M. Demonts. 1999. Methicillin-resistant Staphylococcus aureus as a causative agent of postoperative intra-abdominal infection: relation to nasal colonization. Clin. Infect. Dis. 29:1231-1238.
4. Frebourg, N., B. Cauliez and J.-F. Lemeland. 1999. Evidence for nasal carriage of methicillin-resistant staphylococci colonizing intravascular devices. J. Clin. Micro. 37:1182-1185.
5. Heinrich, P., R. Rosenstein, M. Bohmer, P. Sonner, and F. Gotz 1987. The molecular organization of the lysostaphin gene and its sequences repeated in tandem. Mol. Gen. Genet. 209:563-9.
6. Kline SA, J. de la Harpe , and P. Blackburn. 1994. A colorimetric microtiter plate assay for lysostaphin using a hexaglycine substrate. Anal Biochem. 217:329-31.
7. Kluytmans, J., A. van Belkum and H. Verbrugh. 1997. Nasal carriage of Staphylococcus aureus: epidemiology, underlying mechanisms, and associated risks. Clin. Micro. Rev. 10:505-520.
8. Kluytmans, J. A., J. W. Mouton, M. VandenBergh, M.-J. Manders, A. Maat, J. Wagenvoort, M. Michel and H. Verbrugh. 1996. Reduction of surgical-site infections in cardiothoracic surgery by elimination of nasal carriage of Staphylococcus aureus. Infect. Control.Hosp. Epidem. 17:780-785.
9. Martin, J., F. Perdreau-Remington, M. Kartalija, O. Pasi, M. Webb, J. Gerberding, H. Chambers, M. Tauber and B. Lee. 1999. A randomized clinical trial of mupirocin in the eradication of Staphylococcus aureus nasal carriage in human immunodeficiency virus disease. J. Infect. Dis. 180:896-899.
10. Merkus, F.W., J.C. Verhoef, N.G. Schipper, and E. Marttin. 1999. Cyclodextrins in nasal drug delivery. Advan. Drug Deliv. Rev. 36:41-57.
11. Nakamura, K. et al. 1999. Uptake and Release of Budesonide from Mucoadhesive, pH-sensitive Copolymers and Their Application to Nasal Delivery. J. Control. Release 61:329-335.
12. Natsume, H., S. Iwata, K. Ohtak, M. Miyamoto, M. Yamaguchi, K. Hosoya, and D. Kobayashi. 1999. Screening of cationic compounds as an absorption enhancer for nasal drug delivery. Int. J. Pharma. 185:1-12.
13. Nguyen, M. H., C. Kauffman, R. Goodman, C. Squier, R. Arbeit, N. Singh, M. Wagener and V. Yu. 1999. Nasal carriage of and infection with Staphylococcus aureus in HIV-infected patients. Ann. Int. Med. 130:221-225.
14. Ramkissoon-Ganorkar, C. et al. 1999. Modulating insulin-release profile from pH/thermosensivite polymeric beads through polymer molecular weight. J. Contr. Release 59:287-298.
15. Remington's Pharmaceutical Sciences, 18th Edition (A. Gennaro, ed., Mack Pub., Easton, Pa., 1990).
16. Ribeiro, A.J. et al. 1999. Microencapsulations of lipophilic drugs in chitosan-coated alginate microspheres. Int. J. Pharm. 187:115-123.
17. Sambrook, J., Fritsch, E.F., and Maniatis, T. (1989). Molecular cloning: a laboratory manual, 2nd Edition., Cold Spring Harbour Laboratory Press, Cold Spring Harbour.
18. Schwab, U. E., A. E. Wold, J. L. Carson, M. W. Leigh, P.-W. Cheng, P. H. Gilligan and T. F. Boat. 1993. Increased adherence of Staphylococcus aureus from cystic fibrosis lungs to airway epithelial cells. Am. Rev. Respir. Dis. 148: 365-369.

19. Soane, R.J. et al. 1999. Evaluation of the clearance characteristics of bioadhesive systems in humans. Int. J. Pharm. 178:55-65.
20. Soto, N., A. Vaghjimal, A. Stahl-Avicolli, J. Protic, L. Lutwick and E. Chapnick. 1999. Bacitracin versus mupirocin for Staphylococcus aureus nasal colonization. Infect. Cont. Hosp. Epidem. 20:351-353.
21. Suzuki, Y. and Y. Makino. 1999. Mucosal drug delivery using cellulose derivative as a functional polymer. J. Control. Release. 62:101-107.
22. Takenaga, M., Y. Sirizawa, Y. Azechi, A. Ochiai, Y. Kosaka, R. Igarashi, and Y. Mizushima. 1998. Microparticle resins as a potential nasal drug delivery system for insulin. J. Control. Release. 52:81-87.
23. VadenBergh, M., E. Yzerman, A. Van Belkum, H. Boelens, M. Simmons and H. Verbrugh. 1999. Follow-up of Staphylococcus aureus nasal carriage after 8 years: redefining the persistent carrier state. J. Clin. Micro. 37: 3133-3140.
24. White, A. and J. Smith. 1963. Nasal reservoir as the source of extranasal staphylococci. Antimicrob. Agent. Chem. 3:679-683.
25. Yano, M., Y. Doki, M. Inoue, T. Tsujinaka, H. Shiozaki and M. Monden. 2000. Preoperative intranasal mupirocin ointment significantly reduces postoperative infection with Staphylococcus aureus in patients undergoing upper gastrointestinal surgery. Surg. Today (Japan). 30:16-21.
26. Yu, V. L., A. Goetz, M. Wagener, P. B. Smith, J. D. Rihs, J. Hanchett and J. J. Zuravleff. 1986. Staphylococcus aureus nasal carriage and infection in patients on hemodialysis. New Engl. J. Med. 315:91-96.
27. Zygmunt, W. A. and P. A. Tavormina. 1972. Lysostaphin: Model for a Specific Enzymatic Approach to Infectious Disease. Prog. Drug Res. 16:309-333.
28. Climo, M. W., R. L. Patron, B. P. Goldstein, and G. L. Archer. 1998. Lysostaphin treatment of experimental methicillin-resistant Staphylococcus aureus aortic valve endocarditis. Antimicrob. Agents Chemother. 42:1355-1360.
29. Kokai-Kun, J., T. Chanturiya, and J. Mond. 2002. Lysostaphin as a therapy for systemic Staphylococcus aureus infection. Presented at the American Society for Microbiology 102nd General Meeting, Salt Lake City, Utah.

## Claims

**1.** A homogenous lysostaphin preparation comprising recombinant, truncated lysostaphin, wherein said recombinant truncated lysostaphin lacks the first two 5'-most alanine codons of the wild type mature protein, wherein said recombinant truncated lysostaphin has an N-terminal amino acid sequence selected from the group consisting of "f Met-Thr-His-Glu" and "Thr-His-Glu".

**2.** The homogenous lysostaphin preparation of claim 1, wherein at least 50% of said recombinant truncated lysostaphin possess the N-terminal amino acid sequence "Thr-His-Glu".

**3.** A medicament composition comprising:

a. a homogenous lysostaphin preparation comprising recombinant, truncated lysostaphin, wherein said recombinant truncated lysostaphin lacks the first two 5'-most alanine amino acids of the full length lysostaphin amino acid sequence; and
b. a pharmaceutically acceptable carrier.

wherein said recombinant truncated lysostaphin has an N-terminal amino acid sequence selected from the group consisting of "f-Met-Thr-His-Glu" and "Thr-His-Glu".

**4.** The medicament composition of claim 3, wherein at least 50% of said recombinant truncated lysostaphin possess the N-terminal amino acid sequence "Thr-His-Glu".

**5.** The medicament composition of claim 3, wherein substantially all recombinant, truncated lysostaphin possess the N-terminal amino acid sequence "Thr-His-Glu".

**6.** The homogenous lysostaphin preparation of Claim 1, wherein substantially all recombinant, truncated lysostaphin possess the N-terminal amino acid sequence "Thr-His-Glu".

**7.** The homogenous lysostaphin preparation of Claim 1, wherein said recombinant, truncated lysostaphin possessing the N-terminal amino acid sequence of Thr-His-Glu is fused to a signal sequence, wherein said signal sequence instructs a host bacterial cell to secrete said truncated lysostaphin into the periplasmic space.

## Patentansprüche

1. Homogene Lysostaphin-Zubereitung umfassend rekombinantes, verkürztes Lysostaphin, wobei dem rekombinanten, verkürzten Lysostaphin die ersten zwei ganz äußersten 5'-Alaninkodons des reifen Wildtyp-Proteins fehlen, wobei das rekombinante, verkürzte Lysostaphin eine N-terminale Aminosäuresequenz aufweist, die aus der Gruppe bestehend aus "f-Met-Thr-His-Glu" und "Thr-His-Glu" ausgewählt ist.

2. Homogene Lysostaphin-Zubereitung nach Anspruch 1, wobei mindestens 50% des rekombinanten, verkürzten Lysostaphins die N-terminale Aminosäuresequenz "Thr-His-Glu" besitzt.

3. Arzneimittel-Zusammensetzung umfassend:

   a. eine homogene Lysostaphin-Zubereitung umfassend rekombinantes, verkürztes Lysostaphin, wobei dem rekombinanten, verkürzten Lysostaphin die ersten zwei ganz äußersten 5'-Alaninaminosäuren der Volllängen-Lysostaphin-Aminosäuresequenz fehlen; und
   b. einen pharmazeutisch akzeptablen Träger,

   wobei das rekombinante, verkürzte Lysostaphin eine N-terminale Aminosäuresequenz aufweist, die aus der Gruppe bestehend aus "f-Met-Thr-His-Glu" und "Thr-His-Glu" ausgewählt ist.

4. Arzneimittel-Zusammensetzung nach Anspruch 3, wobei mindestens 50% des rekombinanten, verkürzten Lysostaphins die N-terminale Aminosäuresequenz "Thr-His-Glu" besitzt.

5. Arzneimittel-Zusammensetzung nach Anspruch 3, wobei im Wesentlichen das gesamte rekombinante, verkürzte Lysostaphin die N-terminale Aminosäuresequenz "Thr-His-Glu" besitzt.

6. Homogene Lysostaphin-Zubereitung nach Anspruch 1, wobei im Wesentlichen das gesamte rekombinante, verkürzte Lysostaphin die N-terminale Aminosäuresequenz "Thr-His-Glu" besitzt.

7. Homogene Lysostaphin-Zubereitung nach Anspruch 1, wobei das rekombinante, verkürzte Lysostaphin, das die N-terminale Aminosäuresequenz Thr-His-Glu besitzt, mit einer Signalsequenz fusioniert ist, wobei die Signalsequenz eine Bakterienwirtszelle instruiert, das verkürzte Lysostaphin in den periplasmatischen Raum zu sekretieren.

## Revendications

1. Préparation de lysostaphine homogène comprenant de la lysostaphine tronquée recombinante, dans laquelle ladite lysostaphine tronquée recombinante est dépourvue des deux codons alanine les plus proches de la position 5' de la protéine mature de type sauvage, ladite lysostaphine tronquée recombinante ayant une séquence d'aminoacides N-terminale choisie dans le groupe consistant en "f-Met-Thr-His-Glu" et "Thr-His-Glu".

2. Préparation de lysostaphine homogène suivant la revendication 1, dans laquelle au moins 50 % de ladite lysostaphine tronquée recombinante possèdent la séquence d'aminoacides N-terminale "Thr-His-Glu".

3. Composition de médicament comprenant :

   a. une préparation de lysostaphine homogène comprenant de la lysostaphine tronquée recombinante, dans laquelle ladite lysostaphine tronquée recombinante est dépourvue des deux premiers aminoacides alanine les plus proches de la position 5' de la séquence d'aminoacides de la lysostaphine complète ; et
   b. un support pharmaceutiquement acceptable.

   dans laquelle ladite lysostaphine tronquée recombinante a une séquence d'aminoacides N-terminale choisie dans le groupe consistant en "f-Met-Thr-His-Glu" et "Thr-His-Glu".

4. Composition de médicament suivant la revendication 3, dans laquelle au moins 50 % de ladite lysostaphine tronquée recombinante possèdent la séquence d'aminoacides N-terminale "Thr-His-Glu".

5. Composition de médicament suivant la revendication 3, dans laquelle pratiquement toute la lysostaphine tronquée recombinante possède la séquence d'aminoacides N-terminale "Thr-His-Glu".

6. Préparation de lysostaphine homogène suivant la revendication 1, dans laquelle pratiquement toute la lysostaphine tronquée recombinante possède la séquence d'aminoacides N-terminale "Thr-His-Glu".

7. Préparation de lysostaphine homogène suivant la revendication 1, dans laquelle ladite lysostaphine tronquée recombinante possédant la séquence d'aminoacides N-terminale Thr-His-Glu est fusionnée à une séquence signal, ladite séquence signal amenant une cellule bactérienne hôte à sécréter ladite lysostaphine dans l'espace périplasmique.

## FIGURE 1

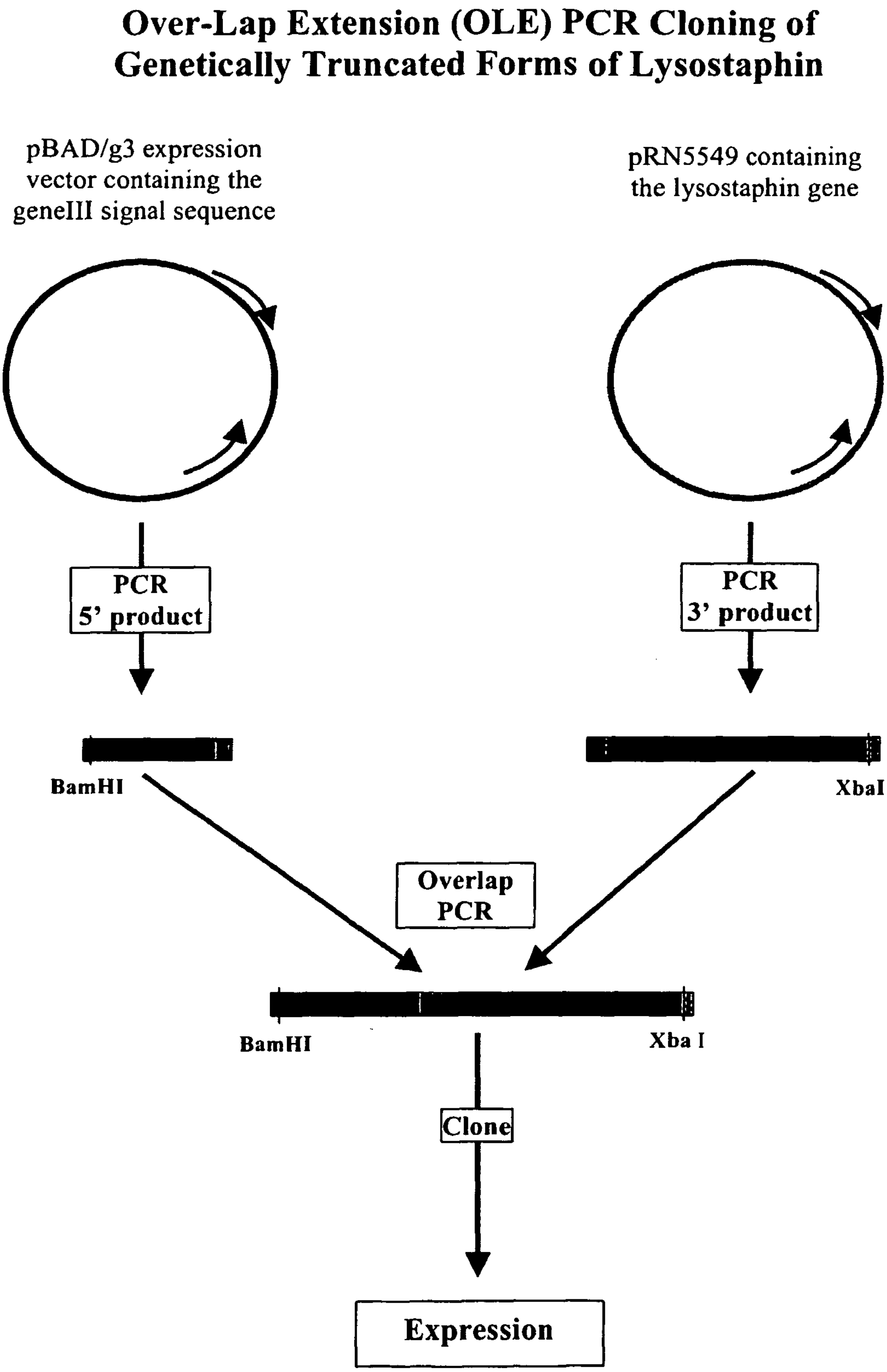
Over-Lap Extension (OLE) PCR Cloning of
Genetically Truncated Forms of Lysostaphin
pBAD/g3 expression
vector containing the
geneIII signal sequence
pRN5549 containing
the lysostaphin gene
PCR
5' product
PCR
3' product
BamHI
XbaI
Overlap
PCR
BamHI
Xba I
Clone
Expression

# FIGURE 2

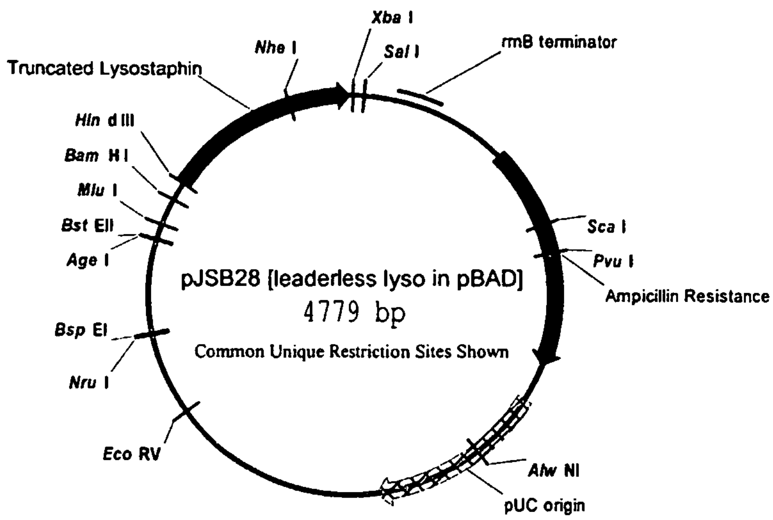


Leaderless Truncated Lysostaphin

```
         M  T  H  E  H  S  A  Q  W  L  N  N  Y  K  K  G  Y  G  Y  G
4041     ATGACACATG AACATTCAGC ACAATGGTTG AATAATTACA AAAAAGGATA TGGTTACGGT
         TACTGTGTAC TTGTAAGTCG TGTTACCAAC TTATTAATGT TTTTTCCTAT ACCAATGCCA
         P  Y  P  L  G  I  N  G  G  M  H  Y  G  V  D  F  F  M  N  I
4101     CCTTATCCAT TAGGTATAAA TGGCGGTATG CACTACGGAG TTGATTTTTT TATGAATATT
         GGAATAGGTA ATCCATATTT ACCGCCATAC GTGATGCCTC AACTAAAAAA ATACTTATAA
         G  T  P  V  K  A  I  S  S  G  K  I  V  E  A  G  W  S  N  Y
4161     GGAACACCAG TAAAAGCTAT TTCAAGCGGA AAAATAGTTG AAGCTGGTTG GAGTAATTAC
         CCTTGTGGTC ATTTTCGATA AAGTTCGCCT TTTTATCAAC TTCGACCAAC CTCATTAATG
         G  G  G  N  Q  I  G  L  I  E  N  D  G  V  H  R  Q  W  Y  M
4221     GGAGGAGGTA ATCAAATAGG TCTTATTGAA AATGATGGAG TGCATAGACA ATGGTATATG
         CCTCCTCCAT TAGTTTATCC AGAATAACTT TTACTACCTC ACGTATCTGT TACCATATAC
         H  L  S  K  Y  N  V  K  V  G  D  Y  V  K  A  G  Q  I  I  G
4281     CATCTAAGTA AATATAATGT TAAAGTAGGA GATTATGTCA AAGCTGGTCA AATAATCGGT
         GTAGATTCAT TTATATTACA ATTTCATCCT CTAATACAGT TTCGACCAGT TTATTAGCCA
         W  S  G  S  T  G  Y  S  T  A  P  H  L  H  F  Q  R  M  V  N
4341     TGGTCTGGAA GCACTGGTTA TTCTACAGCA CCACATTTAC ACTTCCAAAG AATGGTTAAT
         ACCAGACCTT CGTGACCAAT AAGATGTCGT GGTGTAAATG TGAAGGTTTC TTACCAATTA
         S  F  S  N  S  T  A  Q  D  P  M  P  F  L  K  S  A  G  Y  G
4401     TCATTTTCAA ATTCAACTGC CCAAGATCCA ATGCCTTTCT TAAAGAGCGC AGGATATGGA
         AGTAAAAGTT TAAGTTGACG GGTTCTAGGT TACGGAAAGA ATTTCTCGCG TCCTATACCT
         K  A  G  G  T  V  T  P  T  P  N  T  G  W  K  T  N  K  Y  G
4461     AAAGCAGGTG GTACAGTAAC TCCAACGCCG AATACAGGTT GGAAAACAAA CAAATATGGC
         TTTCGTCCAC CATGTCATTG AGGTTGCGGC TTATGTCCAA CCTTTTGTTT GTTTATACCG
         T  L  Y  K  S  E  S  A  S  F  T  P  N  T  D  I  I  T  R  T
4521     ACACTATATA AATCAGAGTC AGCTAGCTTC ACACCTAATA CAGATATAAT AACAAGAACG
         TGTGATATAT TTAGTCTCAG TCGATCGAAG TGTGGATTAT GTCTATATTA TTGTTCTTGC
         T  G  P  F  R  S  M  P  Q  S  G  V  L  K  A  G  Q  T  I  H
4581     ACTGGTCCAT TTAGAAGCAT GCCGCAGTCA GGAGTCTTAA AAGCAGGTCA AACAATTCAT
         TGACCAGGTA AATCTTCGTA CGGCGTCAGT CCTCAGAATT TTCGTCCAGT TTGTTAAGTA
         Y  D  E  V  M  K  Q  D  G  H  V  W  V  G  Y  T  G  N  S  G
4641     TATGATGAAG TGATGAAACA AGACGGTCAT GTTTGGGTAG GTTATACAGG TAACAGTGGC
         ATACTACTTC ACTACTTTGT TCTGCCAGTA CAAACCCATC CAATATGTCC ATTGTCACCG
         Q  R  I  Y  L  P  V  R  T  W  N  K  S  T  N  T  L  G  V  L
4701     CAACGTATTT ACTTGCCTGT AAGAACATGG AATAAATCTA CTAATACTTT AGGTGTTCTT
         GTTGCATAAA TGAACGGACA TTCTTGTACC TTATTTAGAT GATTATGAAA TCCACAAGAA
         W  G  T  I  K          SEQ ID NO: 9
4761     TGGGGAACTA TAAAG       SEQ ID NO: 10
         ACCCCTTGAT ATTTC
```

## FIGURE 3

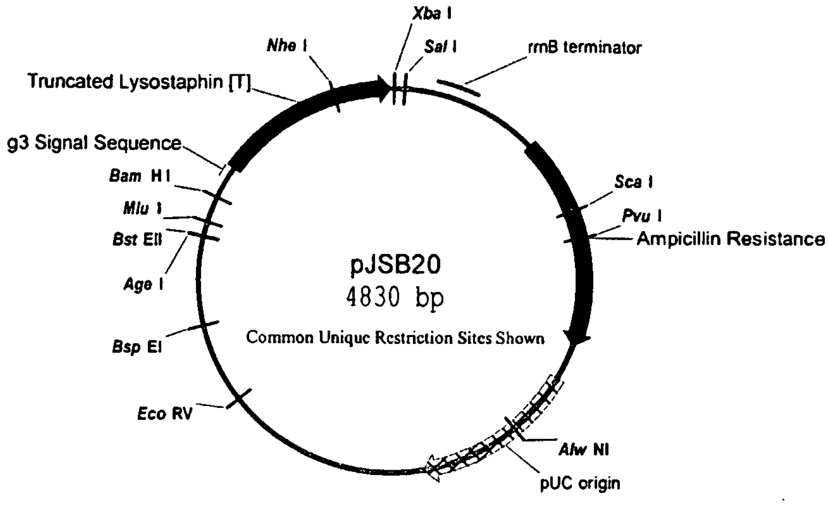


Gene III Signal Sequence and Truncated Lysostaphin

```
        M  K  K  L   L  F  A   I  P  L   V  V  P  F   Y  S  H   S  T  H
4041    ATGAAAAAAC TGCTGTTCGC GATTCCGCTG GTGGTGCCGT TCTATAGCCA TAGCACACAT
        TACTTTTTTG ACGACAAGCG CTAAGGCGAC CACCACGGCA AGATATCGGT ATCGTGTGTA
        E  H  S  A   Q  W  L   N  N  Y   K  K  G  Y   G  Y  G   P  Y  P
4101    GAACATTCAG CACAATGGTT GAATAATTAC AAAAAAGGAT ATGGTTACGG TCCTTATCCA
        CTTGTAAGTC GTGTTACCAA CTTATTAATG TTTTTTCCTA TACCAATGCC AGGAATAGGT
        L  G  I  N   G  G  M   H  Y  G   V  D  F  F   M  N  I   G  T  P
4161    TTAGGTATAA ATGGCGGTAT GCACTACGGA GTTGATTTTT TTATGAATAT TGGAACACCA
        AATCCATATT TACCGCCATA CGTGATGCCT CAACTAAAAA AATACTTATA ACCTTGTGGT
        V  K  A  I   S  S  G   K  I  V   E  A  G  W   S  N  Y   G  G  G
4221    GTAAAAGCTA TTTCAAGCGG AAAAATAGTT GAAGCTGGTT GGAGTAATTA CGGAGGAGGT
        CATTTTCGAT AAAGTTCGCC TTTTTATCAA CTTCGACCAA CCTCATTAAT GCCTCCTCCA
        N  Q  I  G   L  I  E   N  D  G   V  H  R  Q   W  Y  M   H  L  S
4281    AATCAAATAG GTCTTATTGA AAATGATGGA GTGCATAGAC AATGGTATAT GCATCTAAGT
        TTAGTTTATC CAGAATAACT TTTACTACCT CACGTATCTG TTACCATATA CGTAGATTCA
        K  Y  N  V   K  V  G   D  Y  V   K  A  G  Q   I  I  G   W  S  G
4341    AAATATAATG TTAAAGTAGG AGATTATGTC AAAGCTGGTC AAATAATCGG TTGGTCTGGA
        TTTATATTAC AATTTCATCC TCTAATACAG TTTCGACCAG TTTATTAGCC AACCAGACCT
        S  T  G  Y   S  T  A   P  H  L   H  F  Q  R   M  V  N   S  F  S
4401    AGCACTGGTT ATTCTACAGC ACCACATTTA CACTTCCAAA GAATGGTTAA TTCATTTTCA
        TCGTGACCAA TAAGATGTCG TGGTGTAAAT GTGAAGGTTT CTTACCAATT AAGTAAAAGT
        N  S  T  A   Q  D  P   M  P  F   L  K  S  A   G  Y  G   K  A  G
4461    AATTCAACTG CCCAAGATCC AATGCCTTTC TTAAAGAGCG CAGGATATGG AAAAGCAGGT
        TTAAGTTGAC GGGTTCTAGG TTACGGAAAG AATTTCTCGC GTCCTATACC TTTTCGTCCA
        G  T  V  T   P  T  P   N  T  G   W  K  T  N   K  Y  G   T  L  Y
4521    GGTACAGTAA CTCCAACGCC GAATACAGGT TGGAAAACAA ACAAATATGG CACACTATAT
        CCATGTCATT GAGGTTGCGG CTTATGTCCA ACCTTTTGTT TGTTTATACC GTGTGATATA
        K  S  E  S   A  S  F   T  P  N   T  D  I  I   T  R  T   T  G  P
4581    AAATCAGAGT CAGCTAGCTT CACACCTAAT ACAGATATAA TAACAAGAAC GACTGGTCCA
```

```
        TTTAGTCTCA GTCGATCGAA GTGTGGATTA TGTCTATATT ATTGTTCTTG CTGACCAGGT
         F  R  S  M   P  Q  S   G  V  L   K  A  G  Q   T  I  H   Y  D  E
4641    TTTAGAAGCA TGCCGCAGTC AGGAGTCTTA AAAGCAGGTC AAACAATTCA TTATGATGAA
        AAATCTTCGT ACGGCGTCAG TCCTCAGAAT TTTCGTCCAG TTTGTTAAGT AATACTACTT
         V  M  K  Q   D  G  H   V  W  V   G  Y  T  G   N  S  G   Q  R  I
4701    GTGATGAAAC AAGACGGTCA TGTTTGGGTA GGTTATACAG GTAACAGTGG CCAACGTATT
        CACTACTTTG TTCTGCCAGT ACAAACCCAT CCAATATGTC CATTGTCACC GGTTGCATAA
         Y  L  P  V   R  T  W   N  K  S   T  N  T  L   G  V  L   W  G  T
4761    TACTTGCCTG TAAGAACATG GAATAAATCT ACTAATACTT TAGGTGTTCT TTGGGGAACT
        ATGAACGGAC ATTCTTGTAC CTTATTTAGA TGATTATGAA ATCCACAAGA AACCCCTTGA
         I  K         SEQ ID NO: 11
4821    ATAAAG        SEQ ID NO: 12
        TATTTC
```

## FIGURE 4

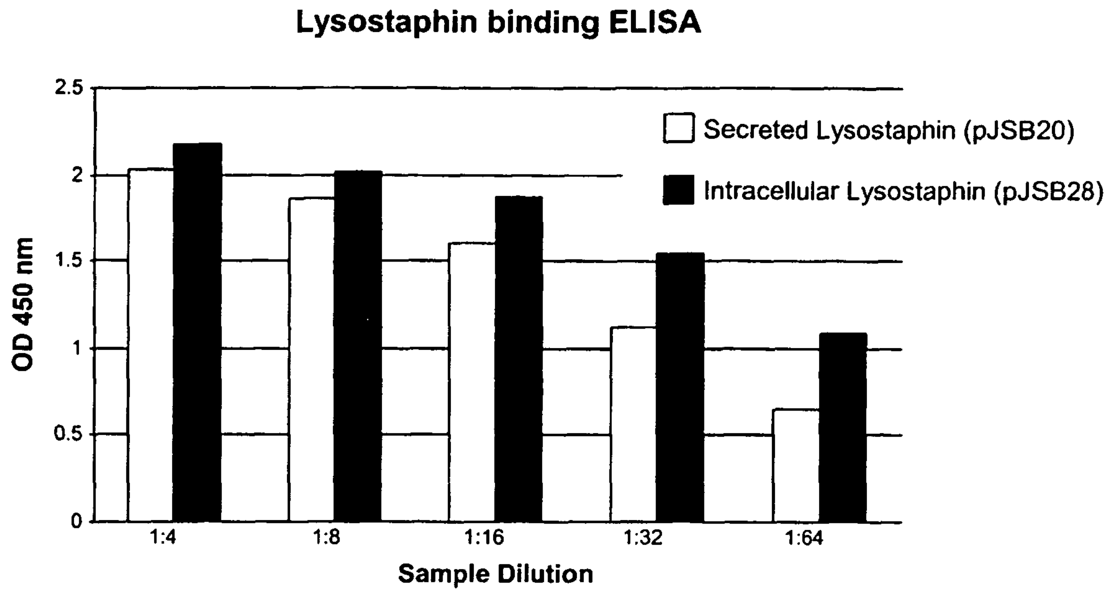
Lysostaphin binding ELISA
2.5
2
1.5
1
0.5
0
OD 450 nm
Secreted Lysostaphin (pJSB20)
Intracellular Lysostaphin (pJSB28)
1:4
1:8
1:16
1:32
1:64
Sample Dilution

## FIGURE 5

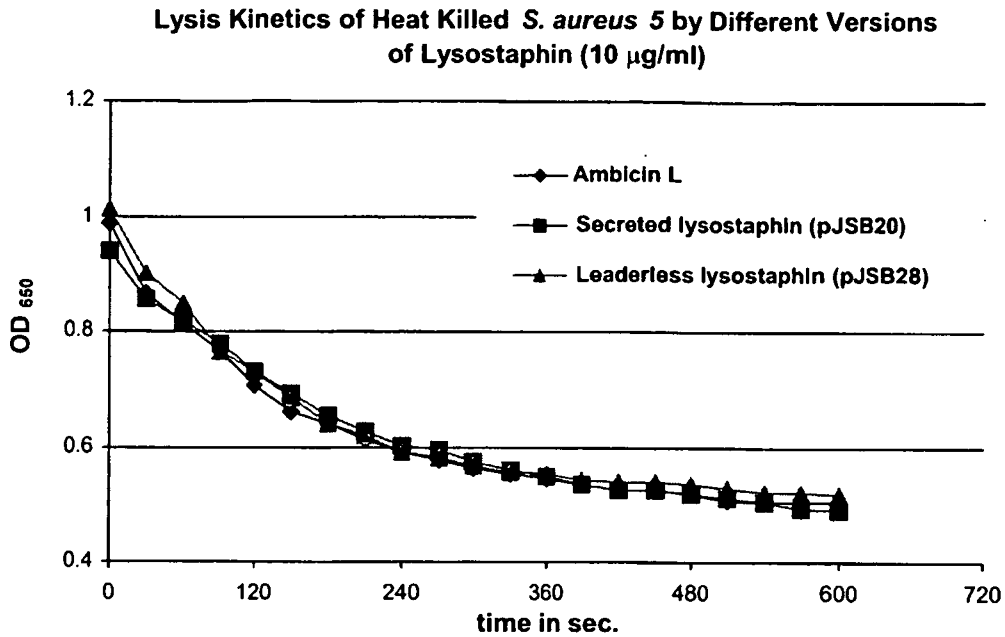
Lysis Kinetics of Heat Killed S. aureus 5 by Different Versions of Lysostaphin (10 μg/ml)
1.2
1
0.8
0.6
0.4
OD 650
0
120
240
360
480
600
720
time in sec.
Ambicin L
Secreted lysostaphin (pJSB20)
Leaderless lysostaphin (pJSB28)

## FIGURE 6A

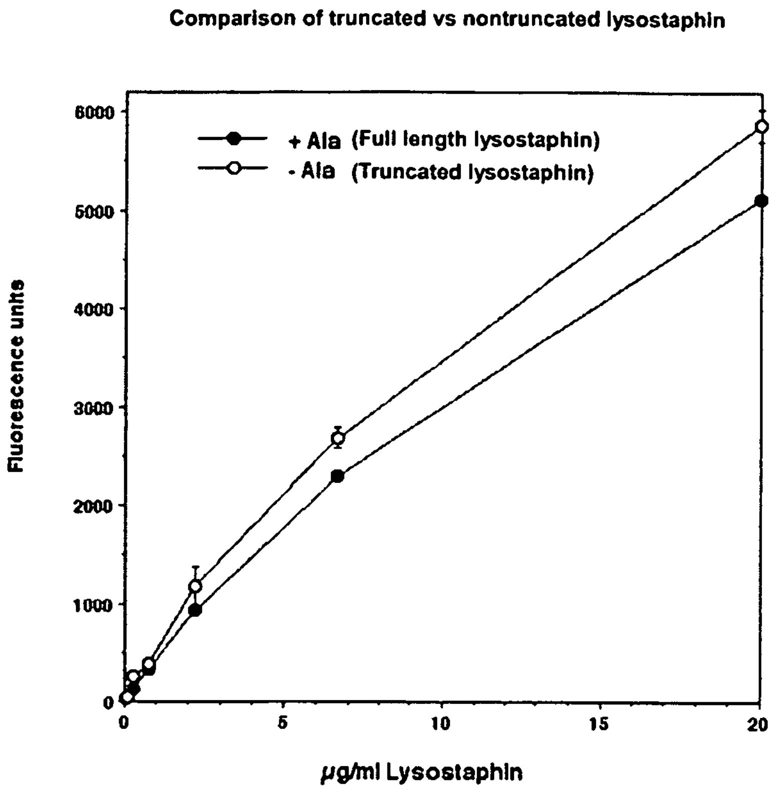
Comparison of truncated vs nontruncated lysostaphin
+ Ala (Full length lysostaphin)
- Ala (Truncated lysostaphin)
Fluorescence units
6000
5000
4000
3000
2000
1000
0
0
5
10
15
20
µg/ml Lysostaphin

FIGURE 6B

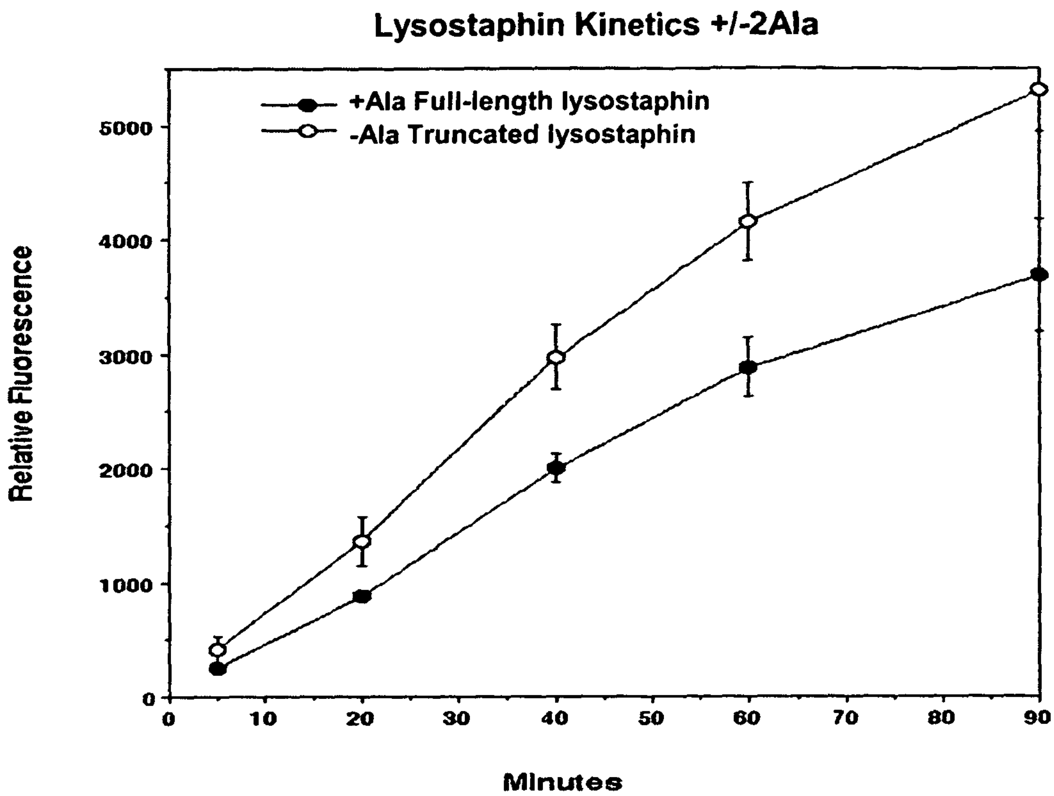
Lysostaphin Kinetics +/-2Ala
+Ala Full-length lysostaphin
-Ala Truncated lysostaphin
Relative Fluorescence
0
1000
2000
3000
4000
5000
0
10
20
30
40
50
60
70
80
90
Minutes

FIGURE 7A

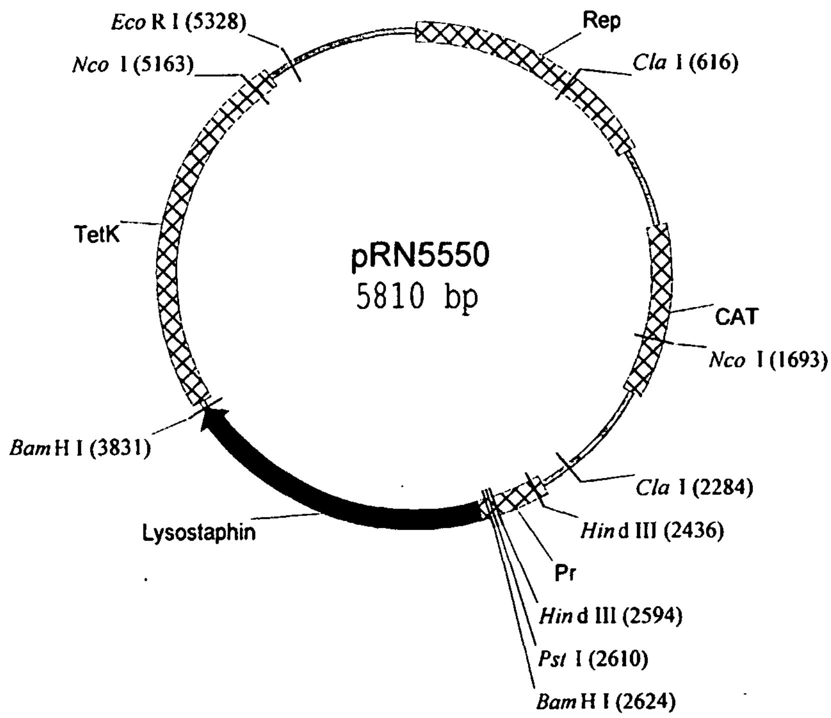
Eco R I (5328)
Nco I (5163)
Rep
Cla I (616)
TetK
pRN5550
5810 bp
CAT
Nco I (1693)
Bam H I (3831)
Cla I (2284)
Lysostaphin
Hin d III (2436)
Pr
Hin d III (2594)
Pst I (2610)
Bam H I (2624)

FIGURE 7B

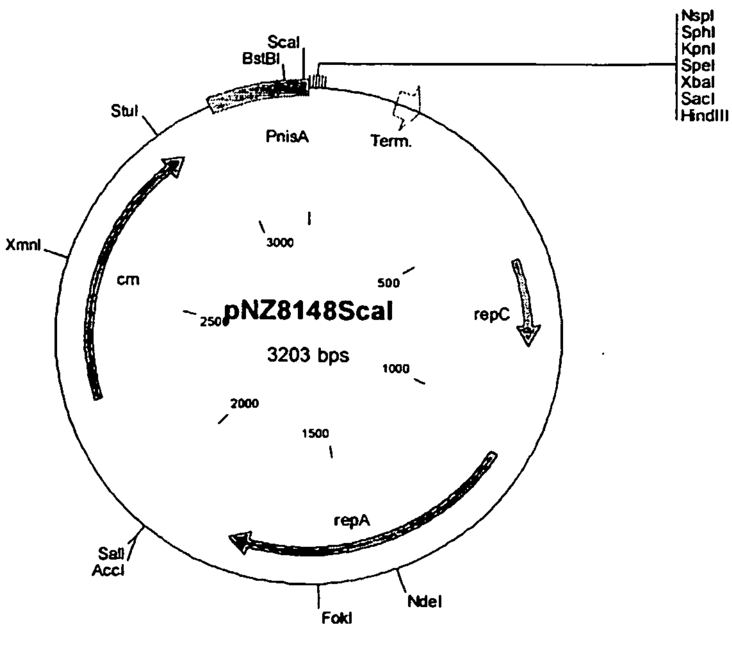
NspI
SphI
KpnI
SpeI
XbaI
SacI
HindIII
ScaI
BstBI
StuI
PnisA
Term.
XmnI
cm
repC
pNZ8148ScaI
3203 bps
3000
500
2500
1000
2000
1500
repA
SalI
AccI
FokI
NdeI

## FIGURE 7C

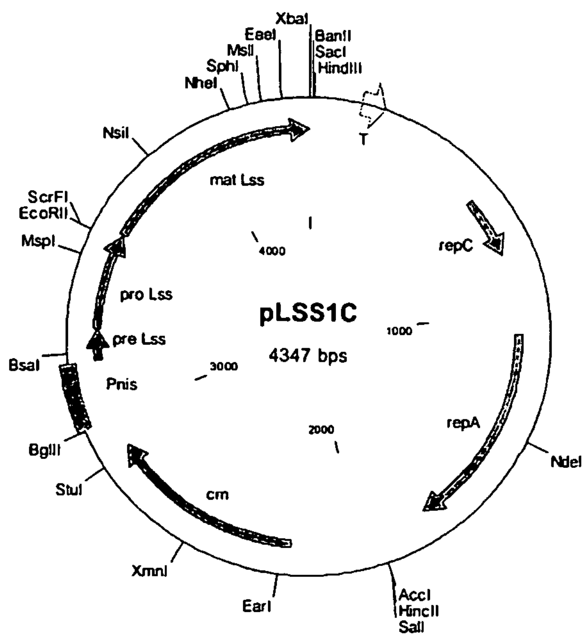
Xbal
Eael
Msll
Sphl
Nhel
BanII
SacI
HindIII
T
Nsil
ScrFI
EcoRII
Mspl
mat Lss
4000
repC
pro Lss
pLSS1C
4347 bps
1000
pre Lss
BsaI
3000
Pnis
2000
repA
Ndel
BglII
StuI
cm
XmnI
EarI
AccI
HincII
SalI

## FIGURE 7D

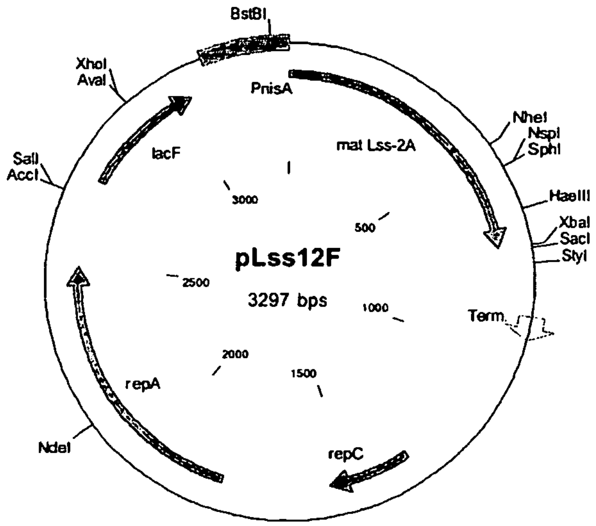
BstBI
XhoI
AvaI
SalI
AccI
PnisA
lacF
mat Lss-2A
NheI
NspI
SphI
HaeIII
XbaI
SacI
StyI
3000
500
pLss12F
3297 bps
2500
1000
Term.
2000
1500
repA
NdeI
repC

# FIGURE 8

## Sequence of Truncated Lysostaphin

T H E H S A Q W L N N Y K K G Y G Y G
ACACATGAACATTCAGCACAATGGTTGAATAATTACAAAAAAGGATATGGTTACGGT

P Y P L G I N G G M H Y G V D F F M N I
CCTTATCCATTAGGTATAAATGGCGGTATGCACTACGGAGTTGATTTTTTTATGAATATT

G T P V K A I S S G K I V E A G W S N Y
GGAACACCAGTAAAAGCTATTTCAAGCGGAAAAATAGTTGAAGCTGGTTGGAGTAATTAC

G G G N Q I G L I E N D G V H R Q W Y M
GGAGGAGGTAATCAAATAGGTCTTATTGAAAATGATGGAGTGCATAGACAATGGTATATG

H L S K Y N V K V G D Y V K A G Q I I G
CATCTAAGTAAATATAACGTTAAAGTAGGAGATTATGTCAAAGCTGGTCAAATAATCGGT

W S G S T G Y S T A P H L H F Q R M V N
TGGTCTGGAAGCACTGGTTATTCTACAGCACCACATTTACACTTCCAAAGAATGGTTAAT

S F S N S T A Q D P M P F L K S A G Y G
TCATTTTCAAATTCAACTGCCCAAGATCCAATGCCTTTCTTAAAGAGCGCAGGATATGGA

K A G G T V T P T P N T G W K T N K Y G
AAAGCAGGTGGTACAGTAACTCCAACGCCGAATACAGGTTGGAAAACAAACAAATATGGC

T L Y K S E S A S F T P N T D I I T R T
ACACTATATAAATCAGAGTCAGCTAGCTTCACACCTAATACAGATATAATAACAAGAACG

T G P F R S M P Q S G V L K A G Q T I H
ACTGGTCCATTTAGAAGCATGCCGCAGTCAGGAGTCTTAAAAGCAGGTCAAACAATTCAT

Y D E V M K Q D G H V W V G Y T G N S G
TATGATGAAGTGATGAAACAAGACGGTCATGTTTGGGTAGGTTATACAGGTAACAGTGGC

```
 Q  R  I  Y  L  P  V  R  T  W  N  K  S  T  N  T  L  G  V  L
CAACGTATTTACTTGCCTGTAAGAACATGGAATAAATCTACTAATACTTTAGGTGTTCTT

 W  G  T  I  K        SEQ ID NO: 13
TGGGGAACTATAAAG       SEQ ID NO: 14
```

**Sequence verified from GenBank entry M15686, with the exception of a silent mutation T → C (Asn → Asn) at position 255*

FIGURE 9

SDS-PAGE reduced

Lyophilized Lysostaphin API

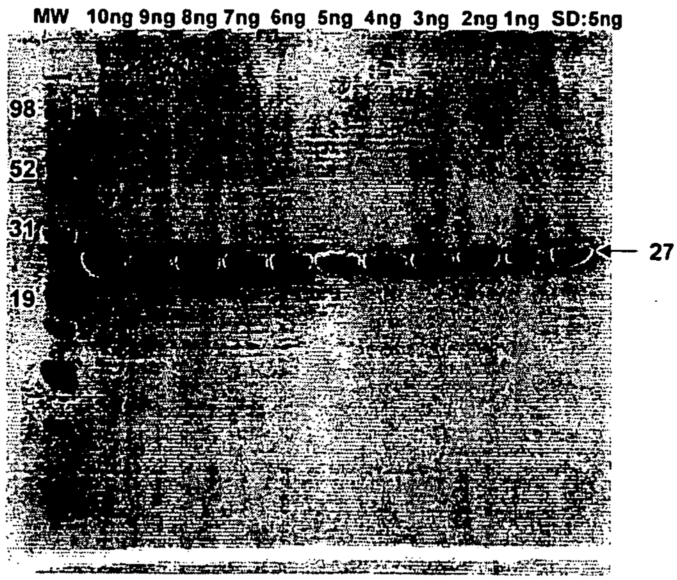

FIGURE 10

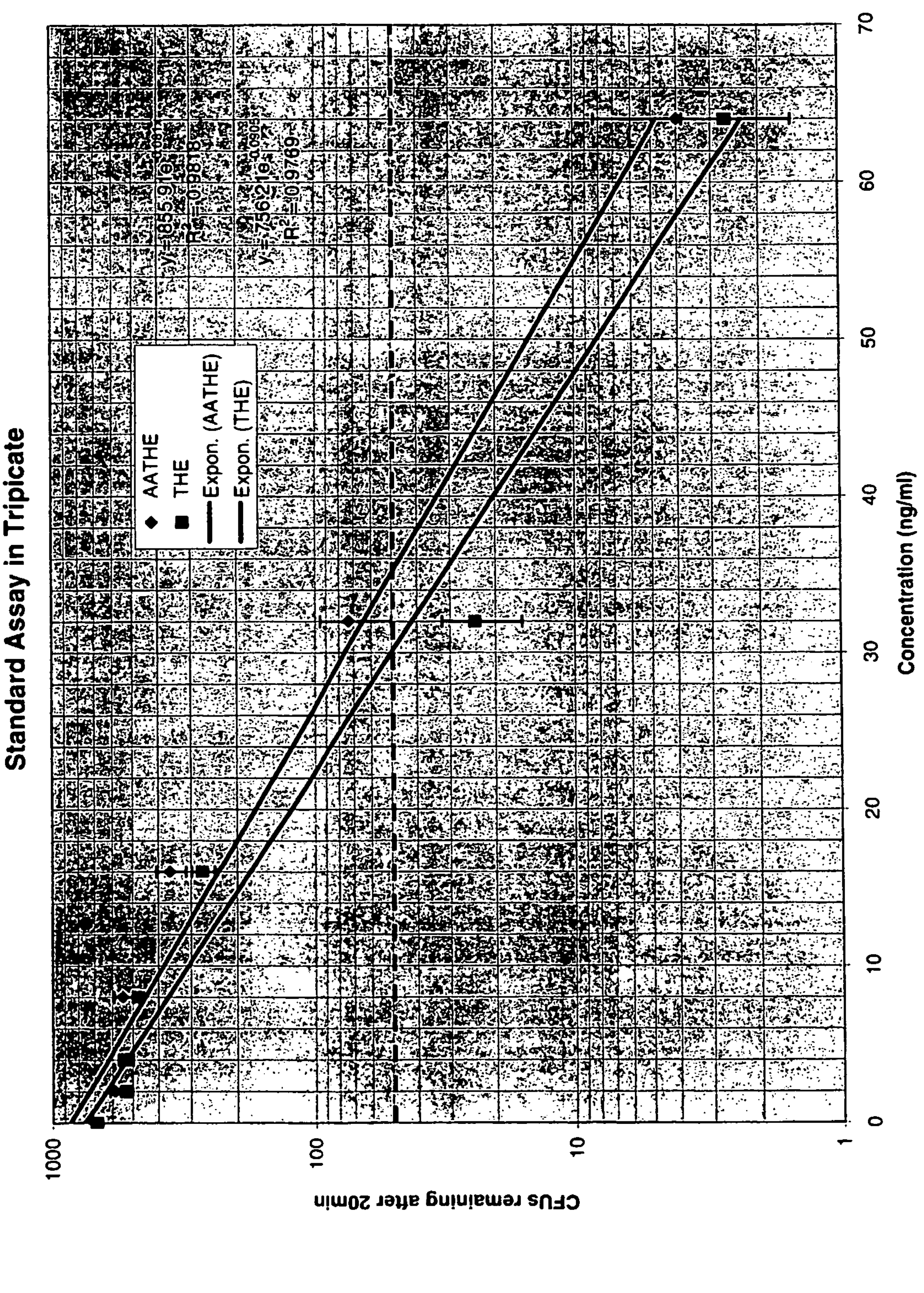
Standard Assay in Tripicate
AATHE
THE
Expon. (AATHE)
Expon. (THE)
CFUs remaining after 20min
1000
100
10
1
Concentration (ng/ml)
0
10
20
30
40
50
60
70

FIGURE 11

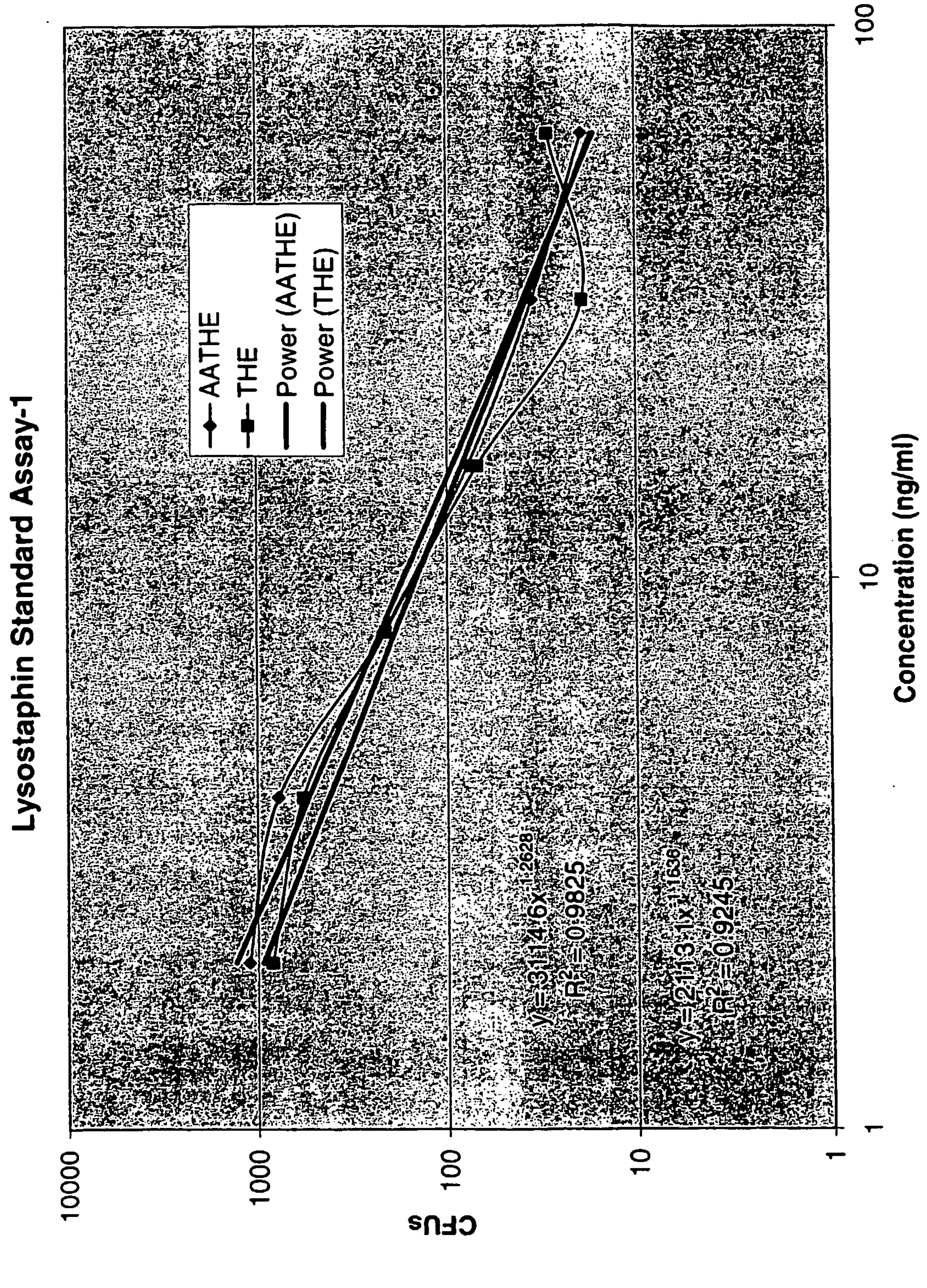

Lysostaphin Standard Assay-1
AATHE
THE
Power (AATHE)
Power (THE)
$y = 3114.6x^{-1.2628}$
$R^2 = 0.9825$
$y = 2113.1x^{-1.1636}$
$R^2 = 0.9245$
CFUs
10000
1000
100
10
1
Concentration (ng/ml)
1
10
100

FIGURE 12

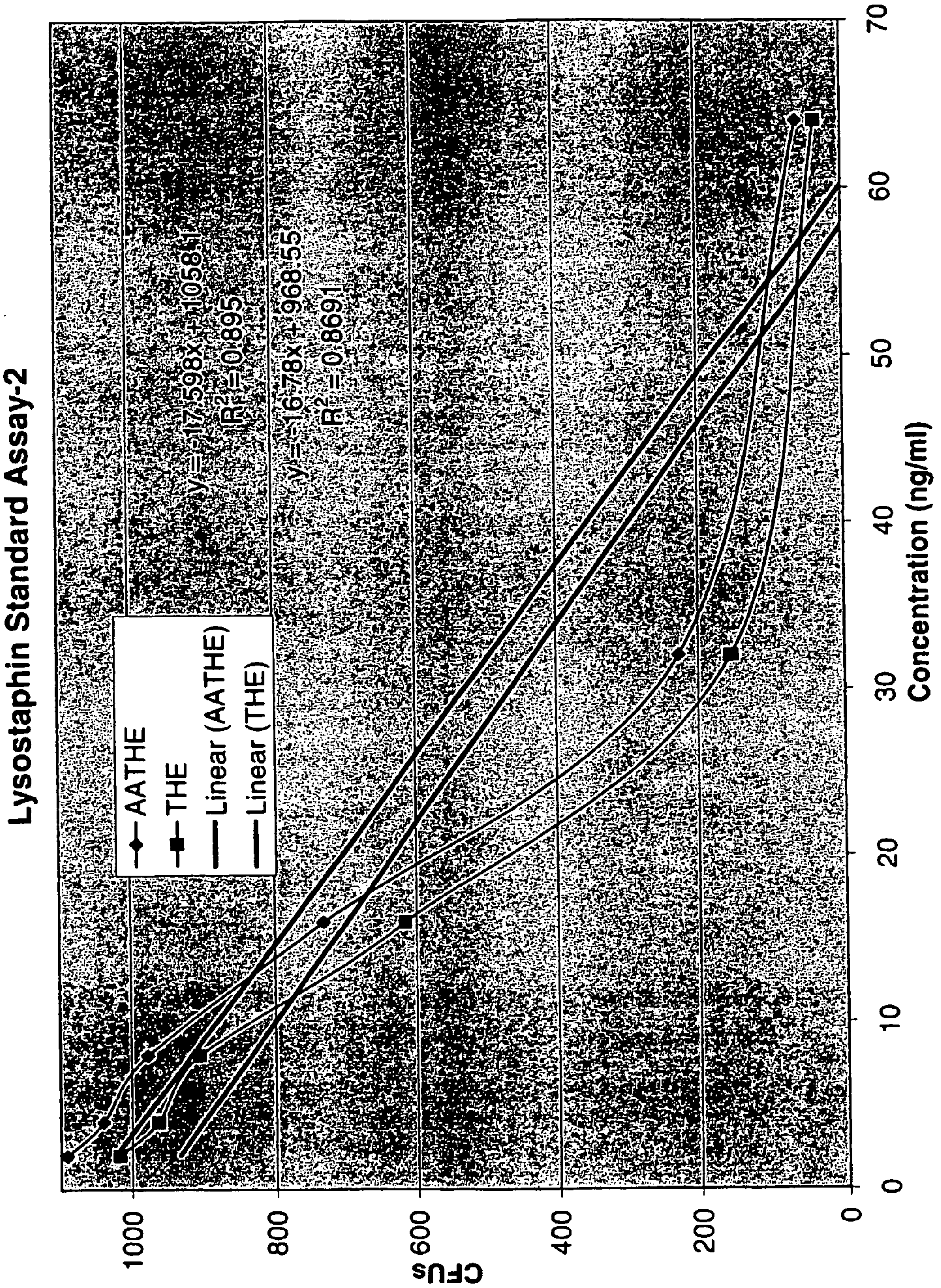
Lysostaphin Standard Assay-2
AATHE
THE
Linear (AATHE)
Linear (THE)
y = -17.598x + 1058.1
R² = 0.895
y = -16.78x + 968.55
R² = 0.8691
CFUs
1000
800
600
400
200
0
0
10
20
30
40
50
60
70
Concentration (ng/ml)

FIGURE 13

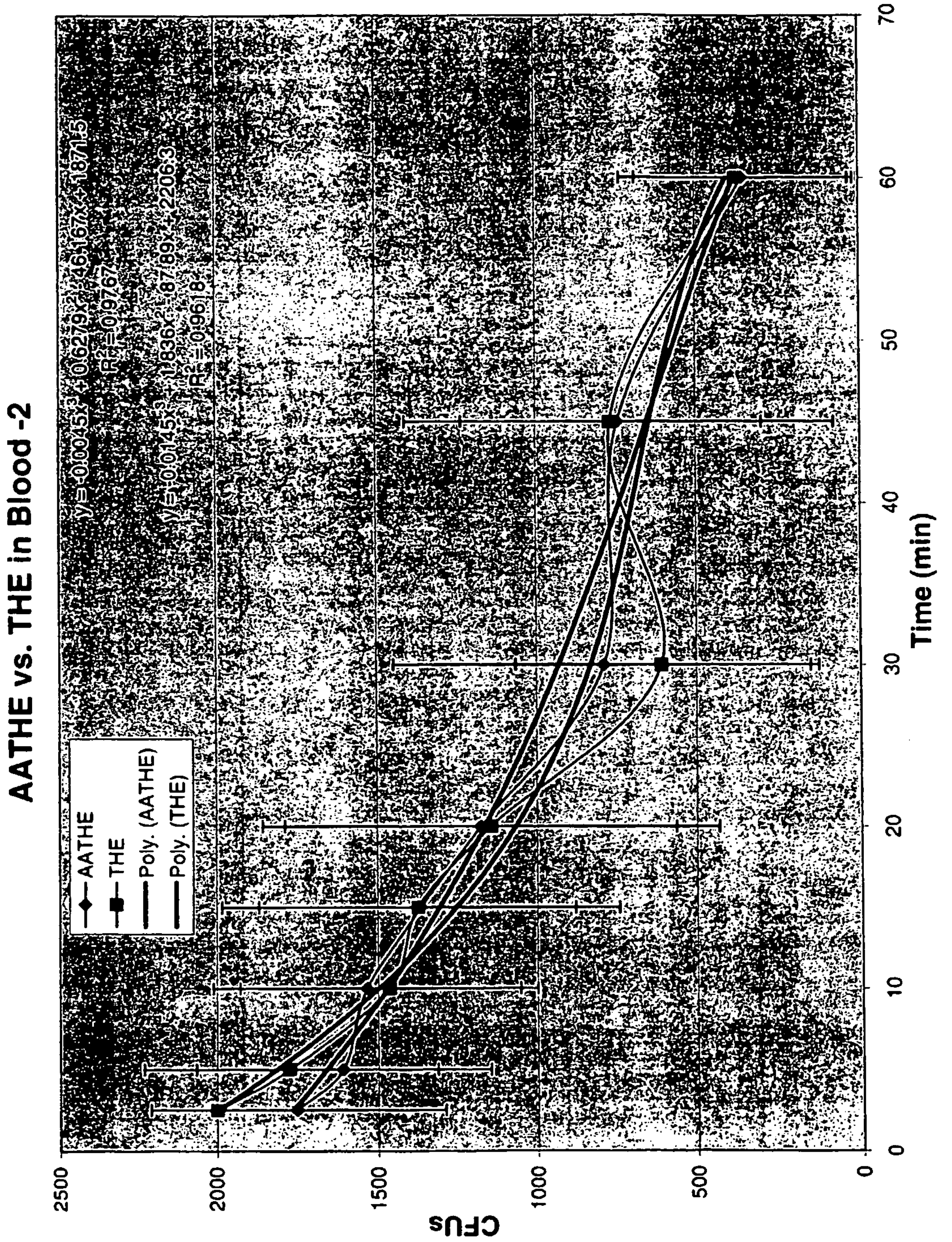
AATHE vs. THE in Blood -2
AATHE
THE
Poly. (AATHE)
Poly. (THE)
CFUs
2500
2000
1500
1000
500
0
Time (min)
0
10
20
30
40
50
60
70

FIGURE 14

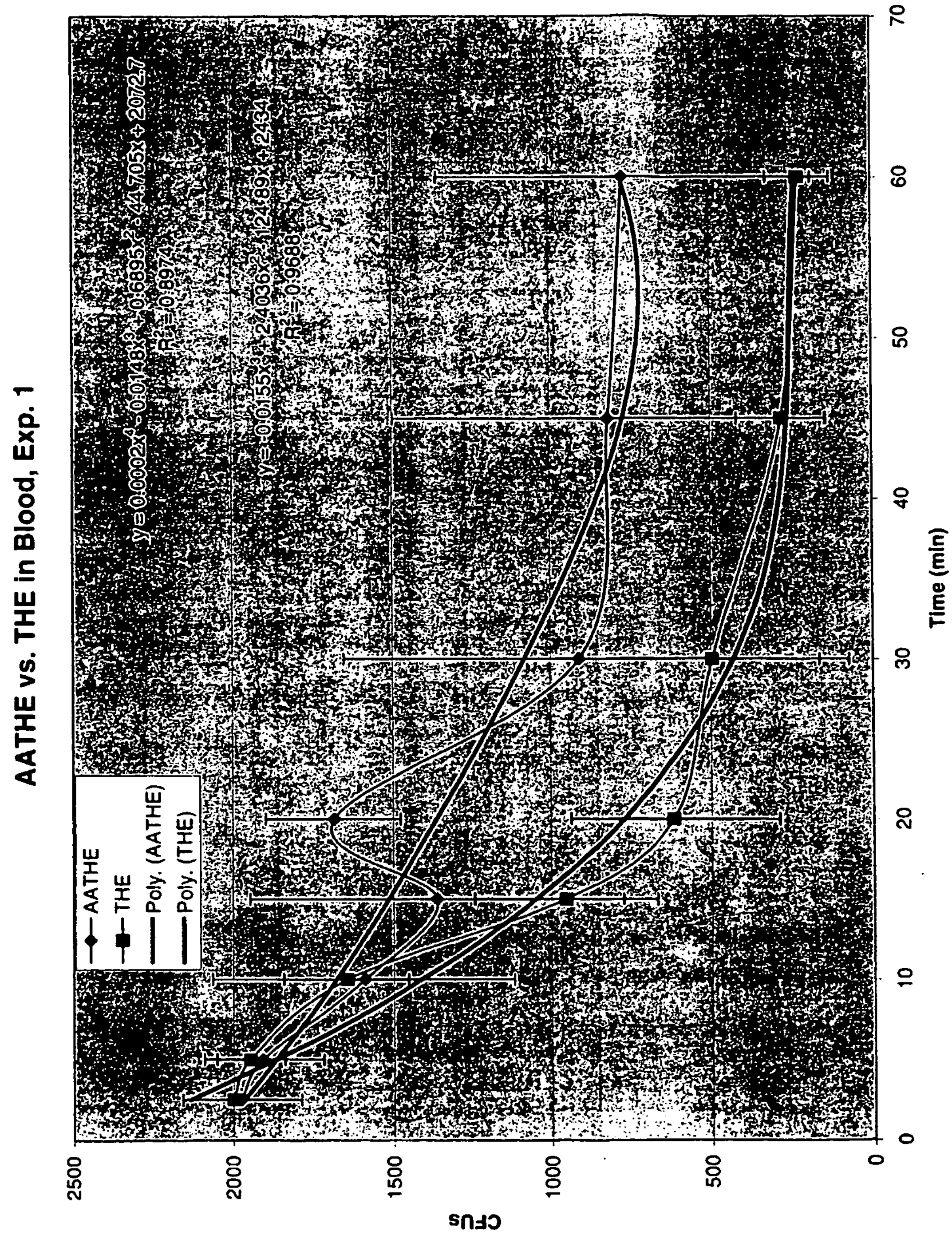
AATHE vs. THE in Blood, Exp. 1
AATHE
THE
Poly. (AATHE)
Poly. (THE)
CFUs
Time (min)
2500
2000
1500
1000
500
0
10
20
30
40
50
60
70

FIGURE 15

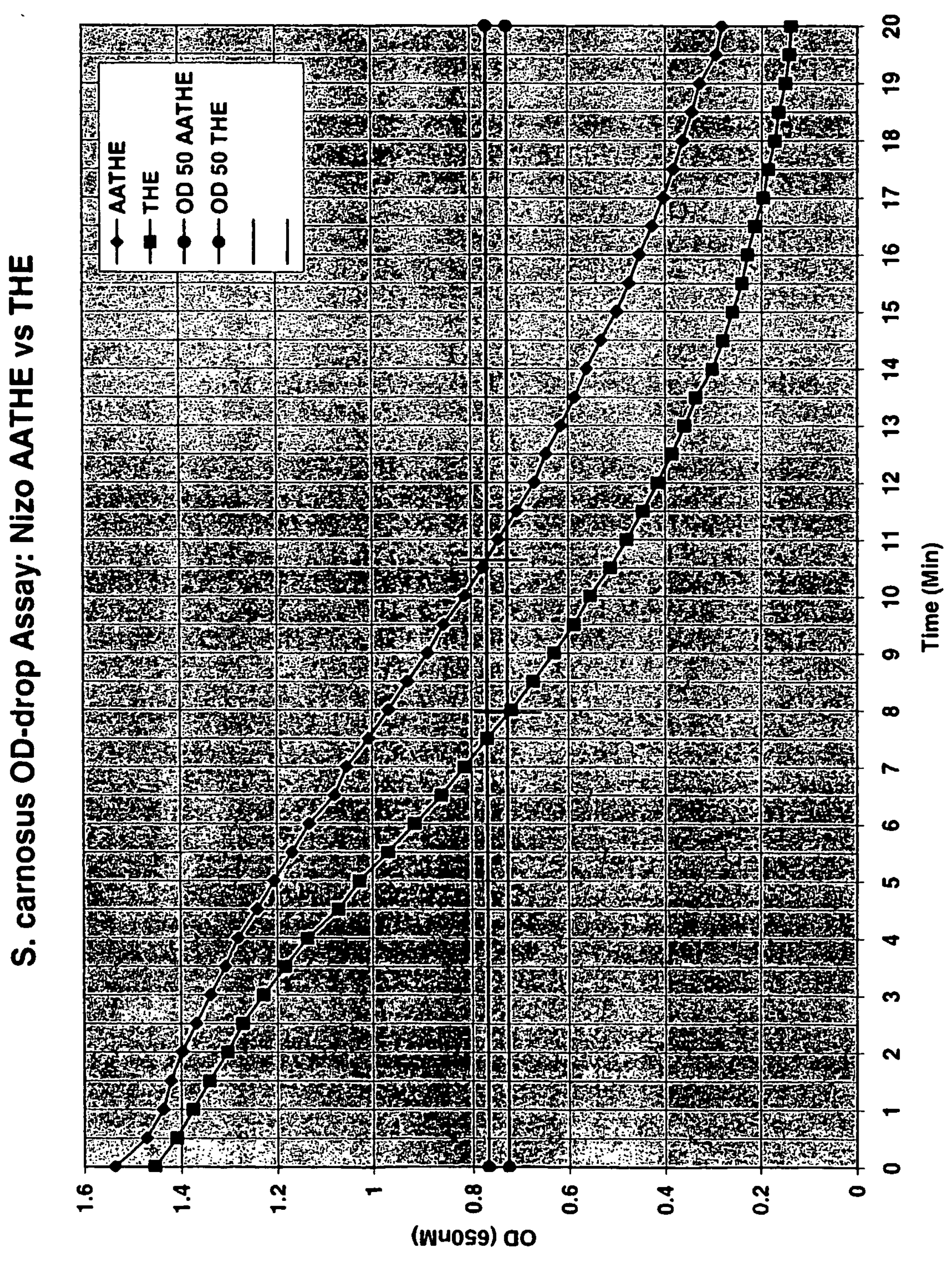
S. carnosus OD-drop Assay: Nizo AATHE vs THE
AATHE
THE
OD 50 AATHE
OD 50 THE
OD (650nM)
Time (Min)
1.6
1.4
1.2
1
0.8
0.6
0.4
0.2
0
0
1
2
3
4
5
6
7
8
9
10
11
12
13
14
15
16
17
18
19
20

FIGURE 16

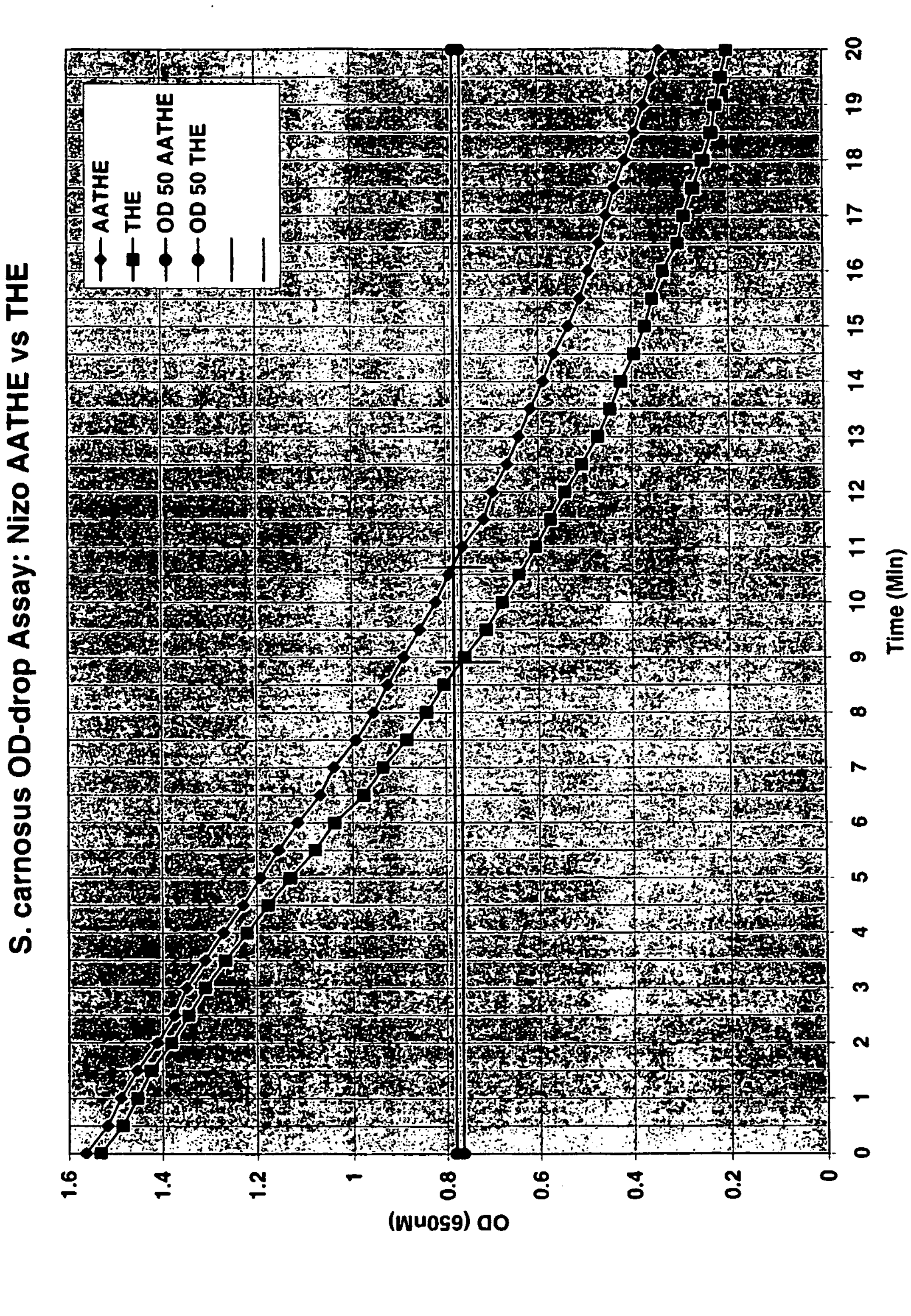
S. carnosus OD-drop Assay: Nizo AATHE vs THE
AATHE
THE
OD 50 AATHE
OD 50 THE
OD (650nM)
Time (Min)
1.6
1.4
1.2
1
0.8
0.6
0.4
0.2
0
0
1
2
3
4
5
6
7
8
9
10
11
12
13
14
15
16
17
18
19
20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 34344401 P
- US 60341804 B


### Non-patent literature cited in the description


- **Chang, F. Y. ; N. Singh ; T. Gayowski ; S. D. Drenning ; M. M. Wagener ; I. R. Marino.** Staphylococcus aureus nasal colonization and association with infections in liver transplant recipients. *Transplantation,* 1998, vol. 65, 1169-1172
- **Chapoutot, C. ; G.-P. Pageaux ; P. F. Perrigault ; Z. Joomaye ; P. Pemey ; H. Jean-Pierre ; O. Jouquet ; P. Blanc ; D. Larrey.** Staphylococcus aureus nasal carriage in 104 cirrhotic and control patients A prospective study. *J. Hepatol.,* 1999, vol. 30, 249-253
- **Fierobe, L. ; D. Decre ; C. Muller ; J.-C. Lucet ; J.-P. Marmuse ; J. Mantz ; J.-M. Demonts.** Methicillin-resistant Staphylococcus aureus as a causative agent of postoperative intra-abdominal infection: relation to nasal colonization. *Clin. Infect. Dis.,* 1999, vol. 29, 1231-1238
- **Frebourg, N. ; B. Cauliez ; J.-F. Lemeland.** Evidence for nasal carriage of methicillin-resistant staphylococci colonizing intravascular devices. *J. Clin. Micro.,* 1999, vol. 37, 1182-1185
- **Heinrich, P. ; R. Rosenstein ; M. Bohmer ; P. Sonner ; F. Gotz.** The molecular organization of the lysostaphin gene and its sequences repeated in tandem. *Mol. Gen. Genet.,* 1987, vol. 209, 563-9
- **Kline SA ; J. de la Harpe ; P. Blackburn.** A colorimetric microtiter plate assay for lysostaphin using a hexaglycine substrate. *Anal Biochem.,* 1994, vol. 217, 329-31
- **Kluytmans, J. ; A. van Belkum ; H. Verbrugh.** Nasal carriage of Staphylococcus aureus: epidemiology, underlying mechanisms, and associated risks. *Clin. Micro. Rev.,* 1997, vol. 10, 505-520
- **Kluytmans, J. A. ; J. W. Mouton ; M. VandenBergh ; M.-J. Manders ; A. Maat ; J. Wagenvoort ; M. Michel ; H. Verbrugh.** Reduction of surgical-site infections in cardiothoracic surgery by elimination of nasal carriage of Staphylococcus aureus. *Infect. Control.Hosp. Epidem.,* 1996, vol. 17, 780-785
- **Martin, J. ; F. Perdreau-Remington ; M. Kartalija ; O. Pasi ; M. Webb ; J. Gerberding ; H. Chambers ; M. Tauber ; B. Lee.** A randomized clinical trial of mupirocin in the eradication of Staphylococcus aureus nasal carriage in human immunodeficiency virus disease. *J. Infect. Dis.,* 1999, vol. 180, 896-899
- **Merkus, F.W. ; J.C. Verhoef ; N.G. Schipper ; E. Marttin.** Cyclodextrins in nasal drug delivery. *Advan. Drug Deliv. Rev.,* 1999, vol. 36, 41-57
- **Nakamura, K. et al.** Uptake and Release of Budesonide from Mucoadhesive, pH-sensitive Copolymers and Their Application to Nasal Delivery. *J. Control. Release,* 1999, vol. 61, 329-335
- **Natsume, H. ; S. Iwata ; K. Ohtak ; M. Miyamoto ; M. Yamaguchi ; K. Hosoya ; D. Kobayashi.** Screening of cationic compounds as an absorption enhancer for nasal drug delivery. *Int. J. Pharma.,* 1999, vol. 185, 1-12
- **Nguyen, M. H. ; C. Kauffman ; R. Goodman ; C. Squier ; R. Arbeit ; N. Singh ; M. Wagener ; V. Yu.** Nasal carriage of and infection with Staphylococcus aureus in HIV-infected patients. *Ann. Int. Med.,* 1999, vol. 130, 221-225
- **Ramkissoon-Ganorkar, C. et al.** Modulating insulin-release profile from pH/thermosensivite polymeric beads through polymer molecular weight. *J. Contr. Release,* 1999, vol. 59, 287-298
- Remington's Pharmaceutical Sciences. Mack Pub, 1990
- **Ribeiro, A.J. et al.** Microencapsulations of lipophilic drugs in chitosan-coated alginate microspheres. *Int. J. Pharm.,* 1999, vol. 187, 115-123
- **Sambrook, J. ; Fritsch, E.F. ; Maniatis, T.** Molecular cloning: a laboratory manual. Cold Spring Harbour Laboratory Press, 1989
- **Schwab, U. E. ; A. E. Wold ; J. L. Carson ; M. W. Leigh ; P.-W. Cheng ; P. H. Gilligan ; T. F. Boat.** Increased adherence of Staphylococcus aureus from cystic fibrosis lungs to airway epithelial cells. *Am. Rev. Respir. Dis.,* 1993, vol. 148, 365-369
- **Soane, R.J. et al.** Evaluation of the clearance characteristics of bioadhesive systems in humans. *Int. J. Pharm.,* 1999, vol. 178, 55-65

- **Soto, N. ; A. Vaghjimal ; A. Stahl-Avicolli ; J. Protic ; L. Lutwick ; E. Chapnick.** Bacitracin versus mupirocin for Staphylococcus aureus nasal colonization. *Infect. Cont. Hosp. Epidem.,* 1999, vol. 20, 351-353
- **Suzuki, Y. ; Y. Makino.** Mucosal drug delivery using cellulose derivative as a functional polymer. *J. Control. Release.,* 1999, vol. 62, 101-107
- **Takenaga, M. ; Y. Sirizawa ; Y. Azechi ; A. Ochiai ; Y. Kosaka ; R. Igarashi ; Y. Mizushima.** Microparticle resins as a potential nasal drug delivery system for insulin. *J. Control. Release.,* 1998, vol. 52, 81-87
- **VadenBergh, M. ; E. Yzerman ; A. Van Belkum ; H. Boelens ; M. Simmons ; H. Verbrugh.** Follow-up of Staphylococcus aureus nasal carriage after 8 years: redefining the persistent carrier state. *J. Clin. Micro.,* 1999, vol. 37, 3133-3140
- **White, A. ; J. Smith.** Nasal reservoir as the source of extranasal staphylococci. *Antimicrob. Agent. Chem.,* 1963, vol. 3, 679-683
- **Yano, M. ; Y. Doki ; M. Inoue ; T. Tsujinaka ; H. Shiozaki ; M. Monden.** Preoperative intranasal mupirocin ointment significantly reduces postoperative infection with Staphylococcus aureus in patients undergoing upper gastrointestinal surgery. *Surg. Today (Japan),* 2000, vol. 30, 16-21
- **Yu, V. L. ; A. Goetz ; M. Wagener ; P. B. Smith ; J. D. Rihs ; J. Hanchett ; J. J. Zuravleff.** Staphylococcus aureus nasal carriage and infection in patients on hemodialysis. *New Engl. J. Med.,* 1986, vol. 315, 91-96
- **Zygmunt, W. A. ; P. A. Tavormina.** Lysostaphin: Model for a Specific Enzymatic Approach to Infectious Disease. *Prog. Drug Res.,* 1972, vol. 16, 309-333
- **Climo, M. W. ; R. L. Patron ; B. P. Goldstein ; G. L. Archer.** Lysostaphin treatment of experimental methicillin-resistant Staphylococcus aureus aortic valve endocarditis. *Antimicrob. Agents Chemother.,* 1998, vol. 42, 1355-1360
- **Kokai-Kun, J. ; T. Chanturiya ; J. Mond.** Lysostaphin as a therapy for systemic Staphylococcus aureus infection. *Presented at the American Society for Microbiology 102nd General Meeting,* 2002

SEQUENCE LISTING

<110> BIOSYNEXUS, INC.

<120> TRUNCATED LYSOSTAPHIN MOLECULE WITH ENHANCED STAPHYLOLYTIC ACTIVITY

<130> 07787.0058-00304

<140>
<141>

<150> 60/341,804
<151> 2001-12-21

<160> 17

<170> Patent In Ver. 2.1

<210> 1
<211> 20
<212> DNA
<213> Artificial Sequence

<220>
<223> Description of Artificial Sequence: Primer

<400> 1
attagcggat cctacctgac 20

<210> 2
<211> 43

<212> DNA
<213> Artificial Sequence

<220>
<223> Description of Artificial Sequence: Primer

<400> 2
ccattgtgct gaatgttcat gtgtgctatg gctatagaac ggc 43

<210> 3
<211> 46
<212> DNA
<213> Artificial Sequence

<220>
<223> Description of Artificial Sequence: Primer

<400> 3
ggtgccgttc tatagccata gcacacatga acattcagca caatgg 46

<210> 4
<211> 37
<212> DNA
<213> Artificial Sequence

<220>
<223> Description of Artificial Sequence: Primer

<400> 4
ttattcttct agatcacttt atagttcccc aaagaac 37

<210> 5
<211> 22
<212> DNA
<213> Artificial Sequence

<220>
<223> Description of Artificial Sequence: Primer

<400> 5
ctatgccata gcatttttat cc 22

<210> 6
<211> 17
<212> DNA
<213> Artificial Sequence

<220>
<223> Description of Artificial Sequence: Primer

<400> 6
agccaagctg gagaccg 17

<210> 7
<211> 44
<212> DNA
<213> Artificial Sequence

<220>
<223> Description of Artificial Sequence: Primer

<400> 7
gggctaacag gaggaagctt ccatgacaca tgaacattca gcac 44

<210> 8
<211> 30
<212> DNA
<213> Artificial Sequence

<220>
<223> Description of Artificial Sequence: Primer

<400> 8
tgtgtcatgg aagcttcctc ctgttagccc 30

<210> 9
<211> 245
<212> PRT
<213> Artificial Sequence

<220>
<223> Description of Artificial Sequence: Amino acid sequence of the truncated lysostaphin protein encoded by pJSB28

<400> 9

```
Met Thr His Glu His Ser Ala Gln Trp Leu Asn Asn Tyr Lys Lys Gly
 1               5                  10                  15

Tyr Gly Tyr Gly Pro Tyr Pro Leu Gly Ile Asn Gly Gly Met His Tyr
            20                  25                  30

Gly Val Asp Phe Phe Met Asn Ile Gly Thr Pro Val Lys Ala Ile Ser
        35                  40                  45

Ser Gly Lys Ile Val Glu Ala Gly Trp Ser Asn Tyr Gly Gly Gly Asn
    50                  55                  60

Gln Ile Gly Leu Ile Glu Asn Asp Gly Val His Arg Gln Trp Tyr Met
65                  70                  75                  80

His Leu Ser Lys Tyr Asn Val Lys Val Gly Asp Tyr Val Lys Ala Gly
                85                  90                  95

Gln Ile Ile Gly Trp Ser Gly Ser Thr Gly Tyr Ser Thr Ala Pro His
            100                 105                 110

Leu His Phe Gln Arg Met Val Asn Ser Phe Ser Asn Ser Thr Ala Gln
        115                 120                 125

Asp Pro Met Pro Phe Leu Lys Ser Ala Gly Tyr Gly Lys Ala Gly Gly
    130                 135                 140

Thr Val Thr Pro Thr Pro Asn Thr Gly Trp Lys Thr Asn Lys Tyr Gly
145                 150                 155                 160

Thr Leu Tyr Lys Ser Glu Ser Ala Ser Phe Thr Pro Asn Thr Asp Ile
                165                 170                 175

Ile Thr Arg Thr Thr Gly Pro Phe Arg Ser Met Pro Gln Ser Gly Val
            180                 185                 190

Leu Lys Ala Gly Gln Thr Ile His Tyr Asp Glu Val Met Lys Gln Asp
        195                 200                 205

Gly His Val Trp Val Gly Tyr Thr Gly Asn Ser Gly Gln Arg Ile Tyr
    210                 215                 220

Leu Pro Val Arg Thr Trp Asn Lys Ser Thr Asn Thr Leu Gly Val Leu
225                 230                 235                 240

Trp Gly Thr Ile Lys
                245
```

<210> 10
<211> 735
<212> DNA
<213> Artificial Sequence

<220>
<223> Description of Artificial Sequence: Nucleotide sequence encoding the truncated lysostaphin protein in pJSB28

<220>
<221> CDS
<222> (1)..(735)

<400> 10

```
atg aca cat gaa cat tca gca caa tgg ttg aat aat tac aaa aaa gga    48
Met Thr His Glu His Ser Ala Gln Trp Leu Asn Asn Tyr Lys Lys Gly
 1               5                  10                  15

tat ggt tac ggt cct tat cca tta ggt ata aat ggc ggt atg cac tac    96
Tyr Gly Tyr Gly Pro Tyr Pro Leu Gly Ile Asn Gly Gly Met His Tyr
            20                  25                  30

gga gtt gat ttt ttt atg aat att gga aca cca gta aaa gct att tca   144
Gly Val Asp Phe Phe Met Asn Ile Gly Thr Pro Val Lys Ala Ile Ser
        35                  40                  45

agc gga aaa ata gtt gaa gct ggt tgg agt aat tac gga gga ggt aat   192
Ser Gly Lys Ile Val Glu Ala Gly Trp Ser Asn Tyr Gly Gly Gly Asn
    50                  55                  60

caa ata ggt ctt att gaa aat gat gga gtg cat aga caa tgg tat atg   240
Gln Ile Gly Leu Ile Glu Asn Asp Gly Val His Arg Gln Trp Tyr Met
65                  70                  75                  80

cat cta agt aaa tat aat gtt aaa gta gga gat tat gtc aaa gct ggt   288
His Leu Ser Lys Tyr Asn Val Lys Val Gly Asp Tyr Val Lys Ala Gly
                85                  90                  95

caa ata atc ggt tgg tct gga agc act ggt tat tct aca gca cca cat   336
Gln Ile Ile Gly Trp Ser Gly Ser Thr Gly Tyr Ser Thr Ala Pro His
            100                 105                 110

tta cac ttc caa aga atg gtt aat tca ttt tca aat tca act gcc caa   384
Leu His Phe Gln Arg Met Val Asn Ser Phe Ser Asn Ser Thr Ala Gln
        115                 120                 125

gat cca atg cct ttc tta aag agc gca gga tat gga aaa gca ggt ggt   432
Asp Pro Met Pro Phe Leu Lys Ser Ala Gly Tyr Gly Lys Ala Gly Gly
    130                 135                 140

aca gta act cca acg ccg aat aca ggt tgg aaa aca aac aaa tat ggc   480
Thr Val Thr Pro Thr Pro Asn Thr Gly Trp Lys Thr Asn Lys Tyr Gly
145                 150                 155                 160
```

```
aca cta tat aaa tca gag tca gct agc ttc aca cct aat aca gat ata     528
Thr Leu Tyr Lys Ser Glu Ser Ala Ser Phe Thr Pro Asn Thr Asp Ile
                165                 170                 175

ata aca aga acg act ggt cca ttt aga agc atg ccg cag tca gga gtc     576
Ile Thr Arg Thr Thr Gly Pro Phe Arg Ser Met Pro Gln Ser Gly Val
            180                 185                 190

tta aaa gca ggt caa aca att cat tat gat gaa gtg atg aaa caa gac     624
Leu Lys Ala Gly Gln Thr Ile His Tyr Asp Glu Val Met Lys Gln Asp
        195                 200                 205

ggt cat gtt tgg gta ggt tat aca ggt aac agt ggc caa cgt att tac     672
Gly His Val Trp Val Gly Tyr Thr Gly Asn Ser Gly Gln Arg Ile Tyr
    210                 215                 220

ttg cct gta aga aca tgg aat aaa tct act aat act tta ggt gtt ctt     720
Leu Pro Val Arg Thr Trp Asn Lys Ser Thr Asn Thr Leu Gly Val Leu
225                 230                 235                 240

tgg gga act ata aag                                                 735
Trp Gly Thr Ile Lys
                245
```

<210> 11
<211> 262
<212> PRT
<213> Artificial Sequence

<220>
<223> Description of Artificial Sequence: Amino acid sequence of the truncated lysostaphin protein and signal polypeptide encoded by pJSB20

<400> 11

```
Met Lys Lys Leu Leu Phe Ala Ile Pro Leu Val Val Pro Phe Tyr Ser
1               5                   10                  15

His Ser Thr His Glu His Ser Ala Gln Trp Leu Asn Asn Tyr Lys Lys
            20                  25                  30

Gly Tyr Gly Tyr Gly Pro Tyr Pro Leu Gly Ile Asn Gly Gly Met His
        35                  40                  45

Tyr Gly Val Asp Phe Phe Met Asn Ile Gly Thr Pro Val Lys Ala Ile
    50                  55                  60

Ser Ser Gly Lys Ile Val Glu Ala Gly Trp Ser Asn Tyr Gly Gly Gly
65                  70                  75                  80

Asn Gln Ile Gly Leu Ile Glu Asn Asp Gly Val His Arg Gln Trp Tyr
                85                  90                  95

Met His Leu Ser Lys Tyr Asn Val Lys Val Gly Asp Tyr Val Lys Ala
            100                 105                 110
```

```
Gly Gln Ile Ile Gly Trp Ser Gly Ser Thr Gly Tyr Ser Thr Ala Pro
        115                 120                 125

His Leu His Phe Gln Arg Met Val Asn Ser Phe Ser Asn Ser Thr Ala
    130                 135                 140

Gln Asp Pro Met Pro Phe Leu Lys Ser Ala Gly Tyr Gly Lys Ala Gly
145                 150                 155                 160

Gly Thr Val Thr Pro Thr Pro Asn Thr Gly Trp Lys Thr Asn Lys Tyr
                165                 170                 175

Gly Thr Leu Tyr Lys Ser Glu Ser Ala Ser Phe Thr Pro Asn Thr Asp
            180                 185                 190

Ile Ile Thr Arg Thr Thr Gly Pro Phe Arg Ser Met Pro Gln Ser Gly
        195                 200                 205

Val Leu Lys Ala Gly Gln Thr Ile His Tyr Asp Glu Val Met Lys Gln
    210                 215                 220

Asp Gly His Val Trp Val Gly Tyr Thr Gly Asn Ser Gly Gln Arg Ile
225                 230                 235                 240

Tyr Leu Pro Val Arg Thr Trp Asn Lys Ser Thr Asn Thr Leu Gly Val
                245                 250                 255

Leu Trp Gly Thr Ile Lys
            260
```

<210> 12
<211> 786
<212> DNA
<213> Artificial Sequence

<220>
<223> Description of Artificial Sequence: Nucleotide sequence encoding the truncated lysostaphin protein and signal polypeptide in pJSB20

<220>
<221> CDS
<222> (1) .. (786)

<400> 12

```
atg aaa aaa ctg ctg ttc gcg att ccg ctg gtg gtg ccg ttc tat agc      48
Met Lys Lys Leu Leu Phe Ala Ile Pro Leu Val Val Pro Phe Tyr Ser
  1               5                  10                  15

cat agc aca cat gaa cat tca gca caa tgg ttg aat aat tac aaa aaa      96
His Ser Thr His Glu His Ser Ala Gln Trp Leu Asn Asn Tyr Lys Lys
             20                  25                  30

gga tat ggt tac ggt cct tat cca tta ggt ata aat ggc ggt atg cac     144
Gly Tyr Gly Tyr Gly Pro Tyr Pro Leu Gly Ile Asn Gly Gly Met His
         35                  40                  45
```

```
tac gga gtt gat ttt ttt atg aat att gga aca cca gta aaa gct att     192
Tyr Gly Val Asp Phe Phe Met Asn Ile Gly Thr Pro Val Lys Ala Ile
     50                  55                  60

tca agc gga aaa ata gtt gaa gct ggt tgg agt aat tac gga gga ggt     240
Ser Ser Gly Lys Ile Val Glu Ala Gly Trp Ser Asn Tyr Gly Gly Gly
 65                  70                  75                  80

aat caa ata ggt ctt att gaa aat gat gga gtg cat aga caa tgg tat     288
Asn Gln Ile Gly Leu Ile Glu Asn Asp Gly Val His Arg Gln Trp Tyr
                 85                  90                  95

atg cat cta agt aaa tat aat gtt aaa gta gga gat tat gtc aaa gct     336
Met His Leu Ser Lys Tyr Asn Val Lys Val Gly Asp Tyr Val Lys Ala
            100                 105                 110

ggt caa ata atc ggt tgg tct gga agc act ggt tat tct aca gca cca     384
Gly Gln Ile Ile Gly Trp Ser Gly Ser Thr Gly Tyr Ser Thr Ala Pro
        115                 120                 125

cat tta cac ttc caa aga atg gtt aat tca ttt tca aat tca act gcc     432
His Leu His Phe Gln Arg Met Val Asn Ser Phe Ser Asn Ser Thr Ala
    130                 135                 140

caa gat cca atg cct ttc tta aag agc gca gga tat gga aaa gca ggt     480
Gln Asp Pro Met Pro Phe Leu Lys Ser Ala Gly Tyr Gly Lys Ala Gly
145                 150                 155                 160

ggt aca gta act cca acg ccg aat aca ggt tgg aaa aca aac aaa tat     528
Gly Thr Val Thr Pro Thr Pro Asn Thr Gly Trp Lys Thr Asn Lys Tyr
                165                 170                 175

ggc aca cta tat aaa tca gag tca gct agc ttc aca cct aat aca gat     576
Gly Thr Leu Tyr Lys Ser Glu Ser Ala Ser Phe Thr Pro Asn Thr Asp
            180                 185                 190

ata ata aca aga acg act ggt cca ttt aga agc atg ccg cag tca gga     624
Ile Ile Thr Arg Thr Thr Gly Pro Phe Arg Ser Met Pro Gln Ser Gly
        195                 200                 205

gtc tta aaa gca ggt caa aca att cat tat gat gaa gtg atg aaa caa     672
Val Leu Lys Ala Gly Gln Thr Ile His Tyr Asp Glu Val Met Lys Gln
    210                 215                 220

gac ggt cat gtt tgg gta ggt tat aca ggt aac agt ggc caa cgt att     720
Asp Gly His Val Trp Val Gly Tyr Thr Gly Asn Ser Gly Gln Arg Ile
225                 230                 235                 240

tac ttg cct gta aga aca tgg aat aaa tct act aat act tta ggt gtt     768
Tyr Leu Pro Val Arg Thr Trp Asn Lys Ser Thr Asn Thr Leu Gly Val
                245                 250                 255

ctt tgg gga act ata aag                                             786
Leu Trp Gly Thr Ile Lys
            260
```

<210> 13
<211> 244
<212> PRT
<213> Artificial Sequence

<220>
<223> Description of Artificial Sequence: Amino acid sequence of the truncated lysostaphin found in the plasmid pLss12F

<400> 13

```
Thr His Glu His Ser Ala Gln Trp Leu Asn Asn Tyr Lys Lys Gly Tyr
1               5                   10                  15

Gly Tyr Gly Pro Tyr Pro Leu Gly Ile Asn Gly Gly Met His Tyr Gly
            20                  25                  30

Val Asp Phe Phe Met Asn Ile Gly Thr Pro Val Lys Ala Ile Ser Ser
        35                  40                  45

Gly Lys Ile Val Glu Ala Gly Trp Ser Asn Tyr Gly Gly Gly Asn Gln
    50                  55                  60

Ile Gly Leu Ile Glu Asn Asp Gly Val His Arg Gln Trp Tyr Met His
65                  70                  75                  80

Leu Ser Lys Tyr Asn Val Lys Val Gly Asp Tyr Val Lys Ala Gly Gln
                85                  90                  95

Ile Ile Gly Trp Ser Gly Ser Thr Gly Tyr Ser Thr Ala Pro His Leu
            100                 105                 110

His Phe Gln Arg Met Val Asn Ser Phe Ser Asn Ser Thr Ala Gln Asp
        115                 120                 125

Pro Met Pro Phe Leu Lys Ser Ala Gly Tyr Gly Lys Ala Gly Gly Thr
    130                 135                 140

Val Thr Pro Thr Pro Asn Thr Gly Trp Lys Thr Asn Lys Tyr Gly Thr
145                 150                 155                 160

Leu Tyr Lys Ser Glu Ser Ala Ser Phe Thr Pro Asn Thr Asp Ile Ile
                165                 170                 175

Thr Arg Thr Thr Gly Pro Phe Arg Ser Met Pro Gln Ser Gly Val Leu
            180                 185                 190

Lys Ala Gly Gln Thr Ile His Tyr Asp Glu Val Met Lys Gln Asp Gly
        195                 200                 205

His Val Trp Val Gly Tyr Thr Gly Asn Ser Gly Gln Arg Ile Tyr Leu
    210                 215                 220

Pro Val Arg Thr Trp Asn Lys Ser Thr Asn Thr Leu Gly Val Leu Trp
225                 230                 235                 240

Gly Thr Ile Lys
```

<210> 14
<211> 732
<212> DNA
<213> Artificial Sequence

<220>
<223> Description of Artificial Sequence: Nucleotide sequence encoding the truncated lysostaphin found in the plasmid pLss12F

<220>
<221> CDS
<222> (1)..(732)

<400> 14

```
aca cat gaa cat tca gca caa tgg ttg aat aat tac aaa aaa gga tat    48
Thr His Glu His Ser Ala Gln Trp Leu Asn Asn Tyr Lys Lys Gly Tyr
 1               5                  10                  15

ggt tac ggt cct tat cca tta ggt ata aat ggc ggt atg cac tac gga    96
Gly Tyr Gly Pro Tyr Pro Leu Gly Ile Asn Gly Gly Met His Tyr Gly
            20                  25                  30

gtt gat ttt ttt atg aat att gga aca cca gta aaa gct att tca agc   144
Val Asp Phe Phe Met Asn Ile Gly Thr Pro Val Lys Ala Ile Ser Ser
        35                  40                  45

gga aaa ata gtt gaa gct ggt tgg agt aat tac gga gga ggt aat caa   192
Gly Lys Ile Val Glu Ala Gly Trp Ser Asn Tyr Gly Gly Gly Asn Gln
    50                  55                  60

ata ggt ctt att gaa aat gat gga gtg cat aga caa tgg tat atg cat   240
Ile Gly Leu Ile Glu Asn Asp Gly Val His Arg Gln Trp Tyr Met His
65                  70                  75                  80

cta agt aaa tat aac gtt aaa gta gga gat tat gtc aaa gct ggt caa   288
Leu Ser Lys Tyr Asn Val Lys Val Gly Asp Tyr Val Lys Ala Gly Gln
                85                  90                  95

ata atc ggt tgg tct gga agc act ggt tat tct aca gca cca cat tta   336
Ile Ile Gly Trp Ser Gly Ser Thr Gly Tyr Ser Thr Ala Pro His Leu
            100                 105                 110

cac ttc caa aga atg gtt aat tca ttt tca aat tca act gcc caa gat   384
His Phe Gln Arg Met Val Asn Ser Phe Ser Asn Ser Thr Ala Gln Asp
        115                 120                 125

cca atg cct ttc tta aag agc gca gga tat gga aaa gca ggt ggt aca   432
Pro Met Pro Phe Leu Lys Ser Ala Gly Tyr Gly Lys Ala Gly Gly Thr
    130                 135                 140

gta act cca acg ccg aat aca ggt tgg aaa aca aac aaa tat ggc aca   480
Val Thr Pro Thr Pro Asn Thr Gly Trp Lys Thr Asn Lys Tyr Gly Thr
145                 150                 155                 160
```

```
cta tat aaa tca gag tca gct agc ttc aca cct aat aca gat ata ata     528
Leu Tyr Lys Ser Glu Ser Ala Ser Phe Thr Pro Asn Thr Asp Ile Ile
                165                 170                 175

aca aga acg act ggt cca ttt aga agc atg ccg cag tca gga gtc tta     576
Thr Arg Thr Thr Gly Pro Phe Arg Ser Met Pro Gln Ser Gly Val Leu
            180                 185                 190

aaa gca ggt caa aca att cat tat gat gaa gtg atg aaa caa gac ggt     624
Lys Ala Gly Gln Thr Ile His Tyr Asp Glu Val Met Lys Gln Asp Gly
        195                 200                 205

cat gtt tgg gta ggt tat aca ggt aac agt ggc caa cgt att tac ttg     672
His Val Trp Val Gly Tyr Thr Gly Asn Ser Gly Gln Arg Ile Tyr Leu
    210                 215                 220

cct gta aga aca tgg aat aaa tct act aat act tta ggt gtt ctt tgg     720
Pro Val Arg Thr Trp Asn Lys Ser Thr Asn Thr Leu Gly Val Leu Trp
225                 230                 235                 240

gga act ata aag                                                     732
Gly Thr Ile Lys
```

<210> 15
<211> 247
<212> PRT
<213> Artificial Sequence

<220>
<223> Description of Artificial Sequence: Illustrative protein sequence

<400> 15

```
Arg Ala Ala Thr His Glu His Ser Ala Gln Trp Leu Asn Asn Tyr Lys
1               5                   10                  15

Lys Gly Tyr Gly Tyr Gly Pro Tyr Pro Leu Gly Ile Asn Gly Gly Met
            20                  25                  30

His Tyr Gly Val Asp Phe Phe Met Asn Ile Gly Thr Pro Val Lys Ala
        35                  40                  45

Ile Ser Ser Gly Lys Ile Val Glu Ala Gly Trp Ser Asn Tyr Gly Gly
    50                  55                  60

Gly Asn Gln Ile Gly Leu Ile Glu Asn Asp Gly Val His Arg Gln Trp
65                  70                  75                  80

Tyr Met His Leu Ser Lys Tyr Asn Val Lys Val Gly Asp Tyr Val Lys
                85                  90                  95

Ala Gly Gln Ile Ile Gly Trp Ser Gly Ser Thr Gly Tyr Ser Thr Ala
            100                 105                 110

Pro His Leu His Phe Gln Arg Met Val Asn Ser Phe Ser Asn Ser Thr
        115                 120                 125
```

```
Ala Gln Asp Pro Met Pro Phe Leu Lys Ser Ala Gly Tyr Gly Lys Ala
    130                 135                 140

Gly Gly Thr Val Thr Pro Thr Pro Asn Thr Gly Trp Lys Thr Asn Lys
145                 150                 155                 160

Tyr Gly Thr Leu Tyr Lys Ser Glu Ser Ala Ser Phe Thr Pro Asn Thr
                165                 170                 175

Asp Ile Ile Thr Arg Thr Thr Gly Pro Phe Arg Ser Met Pro Gln Ser
            180                 185                 190

Gly Val Leu Lys Ala Gly Gln Thr Ile His Tyr Asp Glu Val Met Lys
        195                 200                 205

Gln Asp Gly His Val Trp Val Gly Tyr Thr Gly Asn Ser Gly Gln Arg
    210                 215                 220

Ile Tyr Leu Pro Val Arg Thr Trp Asn Lys Ser Thr Asn Thr Leu Gly
225                 230                 235                 240

Val Leu Trp Gly Thr Ile Lys
                245
```

<210> 16
<211> 248
<212> PRT
<213> Artificial Sequence

<220>
<223> Description of Artificial Sequence: Illustrative protein sequence

<400> 16

```
Leu Arg Ala Ala Thr His Glu His Ser Ala Gln Trp Leu Asn Asn Tyr
1               5                   10                  15

Lys Lys Gly Tyr Gly Tyr Gly Pro Tyr Pro Leu Gly Ile Asn Gly Gly
            20                  25                  30

Met His Tyr Gly Val Asp Phe Phe Met Asn Ile Gly Thr Pro Val Lys
        35                  40                  45

Ala Ile Ser Ser Gly Lys Ile Val Glu Ala Gly Trp Ser Asn Tyr Gly
    50                  55                  60

Gly Gly Asn Gln Ile Gly Leu Ile Glu Asn Asp Gly Val His Arg Gln
65                  70                  75                  80

Trp Tyr Met His Leu Ser Lys Tyr Asn Val Lys Val Gly Asp Tyr Val
                85                  90                  95

Lys Ala Gly Gln Ile Ile Gly Trp Ser Gly Ser Thr Gly Tyr Ser Thr
            100                 105                 110

Ala Pro His Leu His Phe Gln Arg Met Val Asn Ser Phe Ser Asn Ser
        115                 120                 125
```

Thr Ala Gln Asp Pro Met Pro Phe Leu Lys Ser Ala Gly Tyr Gly Lys
130 135 140

Ala Gly Gly Thr Val Thr Pro Thr Pro Asn Thr Gly Trp Lys Thr Asn
145 150 155 160

Lys Tyr Gly Thr Leu Tyr Lys Ser Glu Ser Ala Ser Phe Thr Pro Asn
165 170 175

Thr Asp Ile Ile Thr Arg Thr Thr Gly Pro Phe Arg Ser Met Pro Gln
180 185 190

Ser Gly Val Leu Lys Ala Gly Gln Thr Ile His Tyr Asp Glu Val Met
195 200 205

Lys Gln Asp Gly His Val Trp Val Gly Tyr Thr Gly Asn Ser Gly Gln
210 215 220

Arg Ile Tyr Leu Pro Val Arg Thr Trp Asn Lys Ser Thr Asn Thr Leu
225 230 235 240

Gly Val Leu Trp Gly Thr Ile Lys
245

<210> 17
<211> 246
<212> PRT
<213> Artificial Sequence

<220>
<223> Description of Artificial Sequence: Illustrative protein sequence

<400> 17

Ala Ala Thr His Glu His Ser Ala Gln Trp Leu Asn Asn Tyr Lys Lys
1 5 10 15

Gly Tyr Gly Tyr Gly Pro Tyr Pro Leu Gly Ile Asn Gly Gly Met His
20 25 30

Tyr Gly Val Asp Phe Phe Met Asn Ile Gly Thr Pro Val Lys Ala Ile
35 40 45

Ser Ser Gly Lys Ile Val Glu Ala Gly Trp Ser Asn Tyr Gly Gly Gly
50 55 60

Asn Gln Ile Gly Leu Ile Glu Asn Asp Gly Val His Arg Gln Trp Tyr
65 70 75 80

Met His Leu Ser Lys Tyr Asn Val Lys Val Gly Asp Tyr Val Lys Ala
85 90 95

Gly Gln Ile Ile Gly Trp Ser Gly Ser Thr Gly Tyr Ser Thr Ala Pro
100 105 110

```
His Leu His Phe Gln Arg Met Val Asn Ser Phe Ser Asn Ser Thr Ala
        115                 120                 125

Gln Asp Pro Met Pro Phe Leu Lys Ser Ala Gly Tyr Gly Lys Ala Gly
    130                 135                 140

Gly Thr Val Thr Pro Thr Pro Asn Thr Gly Trp Lys Thr Asn Lys Tyr
145                 150                 155                 160

Gly Thr Leu Tyr Lys Ser Glu Ser Ala Ser Phe Thr Pro Asn Thr Asp
                165                 170                 175

Ile Ile Thr Arg Thr Thr Gly Pro Phe Arg Ser Met Pro Gln Ser Gly
            180                 185                 190

Val Leu Lys Ala Gly Gln Thr Ile His Tyr Asp Glu Val Met Lys Gln
        195                 200                 205

Asp Gly His Val Trp Val Gly Tyr Thr Gly Asn Ser Gly Gln Arg Ile
    210                 215                 220

Tyr Leu Pro Val Arg Thr Trp Asn Lys Ser Thr Asn Thr Leu Gly Val
225                 230                 235                 240

Leu Trp Gly Thr Ile Lys
                245
```